# EUROPÄISCHE PATENTSCHRIFT

(11) **EP 2 696 972 B1**
(45) Veröffentlichungstag und Bekanntmachung des Hinweises auf die Patenterteilung: **24.02.2016**
(21) Anmeldenummer: 12770782.6
(22) Anmeldetag: 13.04.2012
(51) Int. Cl.: B01J 21/00, B01J 23/50, C07D 301/10

(54) **ZINK ENTHALTENDER KATALYSATOR FÜR DIE HERSTELLUNG VON ETHYLENOXID**
ZINC-CONTAINING CATALYST FOR THE PRODUCTION OF ETHYLENE OXIDE
CATALYSEUR CONTENANT DU ZINC, POUR LA PRODUCTION D'OXYDE D'ÉTHYLÈNE

(30) Priorität: 14.04.2011 EP 11162378
(43) Veröffentlichungstag der Anmeldung: 19.02.2014
(73) Patentinhaber: BASF SE, 67056 Ludwigshafen (DE)
(72) Erfinder: ROSENDAHL, Tobias, 68259 Mannheim (DE); MÄURER, Torsten, 67245 Lambsheim (DE); DOBNER, Cornelia, Katharina, 67071 Ludwigshafen (DE); ZÜHLKE, Jürgen, 67346 Speyer (DE)
(86) Internationale Anmeldenummer: PCT/IB2012/051836
(87) Internationale Veröffentlichungsnummer: WO 2012/140616

(56) Entgegenhaltungen:
- US-A- 4 410 453
- US-A1- 2004 050 754
- US-A1- 2008 081 920
- US-A1- 2008 091 038
- US-A1- 2009 177 000
- US-A1- 2010 191 006

## Beschreibung

Die vorliegende Erfindung betrifft ein Verfahren zur Herstellung eines Katalysators für die Herstellung von Ethylenoxid, umfassend Silber und Zink aufgebracht auf einem Träger sowie einen Katalysator, herstellbar oder hergestellt durch dieses Verfahren. Darüber hinaus betrifft die vorliegende Erfindung ein Verfahren zur Herstellung von Ethylenoxid aus Ethylen umfassend eine Oxidation von Ethylen in Gegenwart eines derartigen Katalysators.

Ethylenoxid ist eine wichtige Grundchemikalie und wird industriell häufig durch Direktoxidation von Ethylen mit Sauerstoff in Gegenwart von Silber enthaltenden Katalysatoren hergestellt. Häufig werden Trägerkatalysatoren verwendet, auf die das katalytisch aktive, metallische Silber mittels eines geeigneten Verfahrens aufgebracht wurde. Als Trägermaterial können grundsätzlich verschiedene poröse Materialien, wie z. B. Aktivkohle, Titanoxid, Zirkonoxid oder Siliziumdioxid oder keramische Massen oder Mischungen dieser Materialien eingesetzt werden. In der Regel wird alpha-Aluminiumoxid als Träger verwendet. Beispielhaft für Offenbarungen betreffend die Direktoxidation von Ethen seien die DE-A-2300512, DEA-2521906, EP-A-0014457, DE-A-2454972, EP-A-0172565, EP-A-0357293, EP-A-0266015, EP-A-0011356, EP-A-0085237, DE-C2-2560684 oder die DE-A-2753359 genannt.

Neben Silber als Aktivkomponente enthalten diese Katalysatoren oftmals Promotoren zur Verbesserung der katalytischen Eigenschaften. Beispielhaft seien Alkali- und/oder Erdalkalimetallverbindungen als Promotoren genannt. Einige Schriften lehren die Verwendung von Übergangsmetallen wie Wolfram oder Molybdän. Ein besonders bevorzugter Promotor zur Beeinflussung der Aktivität und/oder Selektivität von Katalysatoren ist Rhenium. Katalysatoren, die Rhenium und/oder andere Übergangsmetallpromotoren in Kombination mit Alkali- und/oder Erdalkalimetallverbindungen enthalten, werden aufgrund ihrer hohen Selektivität bevorzugt industriell eingesetzt. Unter Selektivität ist der molare Prozentsatz an Ethylen, der zu Ethylenoxid reagiert, zu verstehen. Die Aktivität wird durch die Ethylenoxid-Konzentration im Reaktorausgang bei ansonsten konstanten Bedingungen, wie beispielsweise Temperatur, Druck, Gasmenge, Katalysatormenge usw. charakterisiert. Je höher die Ethylenoxid-Konzentration im Reaktorausgangsstrom ist, desto höher ist die Aktivität des Katalysators. Je niedriger die zum Erreichen einer bestimmten Alkylenoxidkonzentration erforderliche Temperatur ist, desto höher ist die Aktivität.

Zum Erreichen einer hohen Selektivität muss die Kombination der Aktivmetalle, sowie die Zusammensetzung des Trägers genau aufeinander abgestimmt werden, um Katalysatoren mit möglichst guten Eigenschaften zu erhalten.

So beschreibt beispielsweise die US 4,410,453 einen Katalysator aus Silber aufgebracht auf einem Zink enthaltenden Träger, wobei Zink in Form von Zinkoxid als Trägerzusatzstoff bei der Herstellung des Trägers verwendet wird. Dabei wird der Zusatz von etwa 4 bis 30 Gew.-% Zink, berechnet als Oxid und bezogen auf das Gesamtgewicht des Trägers, zum Trägerrohmaterial offenbart. Dieser Zusatz führt unter den verwendeten Kalzinationsbedingungen zur Bildung von Zinkspinell. Der Katalysator enthält weder Rhenium noch anderer Promotoren. Die so geformten Katalysatoren zeigen Selektivitäten im Bereich von 60 bis 70%.

Ebenso beschreibt die US 4,007,135 einen Katalysator enthaltend Silber aufgebracht auf einen Träger, wobei zusammen mit dem Silber Metallpromotoren, u.a. Zink auf dem Träger aufgebracht werden.

Ausgehend von diesem Stand der Technik lag eine Aufgabe der vorliegenden Erfindung darin, ein Verfahren zur Herstellung neuer Katalysatoren für die Epoxidation von Ethylen sowie neue Katalysatoren, herstellbar oder hergestellt durch dieses Verfahren, bereitzustellen, die vorteilhafte Aktivitäten und/oder Selektivitäten aufweisen.

Erfindungsgemäß wird diese Aufgabe durch ein Verfahren zur Herstellung eines Katalysators umfassend Silber und Zink, aufgebracht auf einem Träger, und einen Katalysator, hergestellt oder herstellbar durch dieses Verfahren, gelöst, wobei das Verfahren mindestens umfasst
(i) Bereitstellen eines Trägers,
(ii) Aufbringen von Zink auf dem Träger gemäß (i) in einer Menge von 10 ppm bis 1500 ppm, jeweils berechnet als Element und bezogen auf das Gesamtgewicht des Trägers gemäß (i), durch Inkontaktbringen des Trägers mit mindestens einem Gemisch G1 umfassend mindestens eine Zinkverbindung,
(iii) Trocknen und/oder Calcinieren des gemäß (ii) erhaltenen Trägers,
(iv) Aufbringen von Silber auf dem getrockneten und/oder calcinierten Träger durch Inkontaktbringen des Trägers mit mindestens einem Gemisch G2 umfassend mindestens eine Silberverbindung.

Weiterhin beschreibt die Erfindung einen Katalysator zur Herstellung von Ethylenoxid an sich, wobei der Katalysator einen Träger und Silber und Zink aufgebracht auf diesen Träger umfasst, wobei Zink in einer Menge von 10 ppm bis 1500 ppm, jeweils berechnet als Element und bezogen auf das Gesamtgewicht des Trägers, auf dem Träger aufgebracht wurde.

Überraschend wurde gefunden, dass Zink enthaltende Katalysatoren, in denen 10 ppm bis 1500 ppm Zink auf dem Träger aufgebracht wurden und in denen das Zink nicht zusammen mit Silber sondern in einem separaten Schritt, insbesondere einem vorgelagerten Schritt auf den Träger aufgebracht wurde, besonders vorteilhafte Eigenschaften zeigen. Insbesondere zeigen die Katalysatoren vorteilhafte Startselektivitäten und/oder Selektivitäten bei der Herstellung von Ethylenoxid. Unter dem Begriff "Startselektivität" wie er im Rahmen der vorliegenden Erfindung verwendet wird, ist der molare Prozentsatz an Ethylen, der in den ersten 80 h Laufzeit zu Ethylenoxid umgesetzt wird, zu verstehen.

### Schritt (i)

Beispiele für geeignete Materialien für den in Schritt (i) des erfindungsgemäßen Verfahrens bereitgestellten Träger sind Aluminiumoxid, Siliziumdioxid, Siliziumcarbid, Titandioxid, Zirkoniumdioxid und Mischungen daraus, wobei Aluminiumoxid bevorzugt ist.

Gemäß einer bevorzugten Ausführungsform betrifft die vorliegende Erfindung demnach ein Verfahren, wie oben beschrieben, bzw. einen Katalysator, herstellbar oder hergestellt durch dieses Verfahren, wobei der Träger ein Aluminiumoxidträger ist. Weiterhin beschreibt die vorliegende Erfindung auch einen wie oben beschriebenen Katalysator an sich, wobei der Träger ein Aluminiumoxidträger ist.

Der Begriff Aluminiumoxid, wie vorliegend verwendet, umfasst alle denkbaren Strukturen wie alpha-, gamma- oder tetha-Aluminiumoxid. Gemäß einer bevorzugten Ausführungsform ist der Träger ein alpha-Aluminiumoxidträger.

Gemäß einer weiter bevorzugten Ausführungsform hat das alpha-Aluminiumoxid eine Reinheit von mindestens 75 %, bevorzugt eine Reinheit von mindestens 80 %, weiter bevorzugt eine Reinheit von mindestens 85 %, weiter bevorzugt eine Reinheit von mindestens 90 %. Beispielsweise hat das alpha-Aluminumoxid eine Reinheit von mindestens 98 %, von mindestens 98,5 % oder von mindestens 99 %.

Der Begriff alpha-Aluminiumoxid umfasst demnach auch alpha-Aluminiumoxide, die weitere Bestandteile enthalten, insbesondere Bestandteile ausgewählt aus der Gruppe bestehend aus Zirkon, Alkalimetallen, Erdalkalimetallen, Silizium, Zink, Gallium, Hafnium, Bor, Fluor, Kupfer, Nickel, Mangan, Eisen, Cer, Titan, Chrom und Gemischen aus zwei oder mehreren davon.

Das alpha-Aluminiumoxid kann dabei die Bestandteile in jeder geeigneten Form enthalten, beispielsweise als Element oder in Form einer oder mehrere Verbindungen. Enthält das alpha-Aluminiumoxid dabei ein oder mehrere Bestandteile in Form einer Verbindung, so enthält es diesen oder diese beispielsweise als Oxid oder Mischoxid.

Was die Menge der weiteren Bestandteile betrifft, so liegt der Gesamtgehalt der weiteren Bestandteile bevorzugt in einem Bereich von weniger als 25 Gew.-%, weiter bezogen weniger als 20 %, weiter bevorzugt weniger als 15 Gew.-% und weiter bevorzugt weniger als 10 Gew.-%, bezogen auf das Gesamtgewicht des Trägers, und berechnet als Summe der sich von Aluminium und Sauerstoff unterscheidenden Elemente.

Enthält der Träger beispielsweise Silicium, so enthält es dieses bevorzugt in einer Menge im Bereich von 50 bis 10000 ppm, weiter bevorzugt in einer Menge von 100 bis 5000 ppm, weiter bevorzugt in einer Menge von 1000 bis 2800 ppm, bezogen auf das Gesamtgewicht des Trägers und berechnet als Element.

Gemäß einer besonders bevorzugten Ausführungsform der Erfindung, ist der Träger ein alpha-Aluminiumoxidträger, wobei das alpha-Aluminiumoxid eine Reinheit von mindestens 85 % hat und Silicium in einer Menge im Bereich von 50 bis 10000 ppm, weiter bevorzugt in einer Menge von 100 bis 5000 ppm, und weiter bevorzugt in einer Menge von 1000 bis 2800 ppm, enthält.

Enthält der Träger beispielsweise Alkalimetalle, so enthält es diese bevorzugt in einer Gesamtmenge im Bereich von höchstens 2500 ppm, weiter bevorzugt in einer Gesamtmenge von 10 bis 1500 ppm, weiter bevorzugt in einer Gesamtmenge von 50 bis 1000 ppm, bezogen auf das Gesamtgewicht des Trägers und berechnet als Element.

Gemäß einer bevorzugten Ausführungsform enthält der Träger mindestens ein Alkalimetall, insbesondere Natrium und/oder Kalium.

Demnach betrifft die vorliegende Erfindung auch ein Verfahren, wie oben beschrieben, und einen Katalysator, herstellbar oder hergestellt durch dieses Verfahren, wobei der Träger mindestens ein Alkalimetall, insbesondere Natrium und/oder Kalium enthält. Weiterhin beschreibt die vorliegende Erfindung auch einen wie oben beschriebenen Katalysator an sich, wobei der Träger mindestens ein Alkalimetall, insbesondere Natrium und/oder Kalium, enthält.

Enthält der Träger Natrium, so enthält es dieses bevorzugt in einer Menge im Bereich von 10 bis 1500 ppm, weiter bevorzugt in einer Menge von 10 bis 1000 ppm, weiter bevorzugt in einer Menge von 10 bis 800 ppm, weiter bevorzugt in einer Menge von 10 bis 500 ppm, bezogen auf das Gesamtgewicht des Trägers und berechnet als Element, wobei die Gesamtmenge aller Alkalimetalle, wie oben beschrieben, bevorzugt im Bereich von 10 bis 2500 ppm liegt.

Enthält der Träger Kalium, so enthält es dieses bevorzugt in einer Menge von höchsten 1000 ppm, weiter bevorzugt in einer Menge von höchstens 500 ppm, weiter bevorzugt in einer Menge von höchstens 200 ppm, beispielsweise im Bereich von 10 bis 200 ppm, bezogen auf das Gesamtgewicht des Trägers und berechnet als Element, wobei die Gesamtmenge aller Alkalimetalle, wie oben beschrieben, bevorzugt im Bereich von 10 bis 2500 ppm liegt.

Gemäß einer bevorzugten Ausführungsform der Erfindung enthält der Träger Natrium in einer Menge von 10 bis 1500 ppm und Kalium in einer Menge von höchstens 1000 ppm. Gemäß einer weiteren Ausführungsform enthält der Träger mindestens ein Erdalkalimetall. Enthält der Träger mindestens ein Erdalkalimetall, so enthält er bevorzugt zusätzlich mindestens ein Alkalimetall, wie vorstehend beschrieben.

Enthält der Träger mindestens ein Erdalkalimetall, so enthält er bevorzugt eine Gesamtmenge an Erdalkalimetallen im Bereich von höchstens 2500 ppm, beispielsweise im Bereich von 1 bis 2500 ppm, weiter bevorzugt in einer Menge von 10 bis 1200 ppm, weiter bevorzugt in einer Menge von 100 bis 800 ppm, bezogen auf das Gesamtgewicht des Trägers und berechnet als Element. Der Begriff "Gesamtmenge an Erdalkalimetallen", wie vorliegend verwendet, bezieht sich auf die Summe aller gegebenenfalls im Träger enthaltenen Erdalkalimetalle, bezogen auf das Gesamtgewicht des Trägers und berechnet als Element.

Gemäß einer Ausführungsform der Erfindung enthält der Träger mindestens ein Erdalkalimetall ausgewählt aus der Gruppe bestehend aus Calcium und Magnesium.

Enthält der Träger beispielsweise Calcium, so enthält es dieses bevorzugt in einer Menge im Bereich von 10 bis 1500 ppm, weiter bevorzugt in einer Menge von 20 bis 1000 ppm, weiter bevorzugt in einer Menge von 30 bis 500 ppm, bezogen auf das Gesamtgewicht des Trägers und berechnet als Element.

Enthält der Träger beispielsweise Magnesium, so enthält es dieses bevorzugt in einer Menge im Bereich von höchstens 800 ppm, bevorzugt in einer Menge von 1 bis 500 ppm, weiter bevorzugt in einer Menge von 1 bis 250 ppm, weiter bevorzugt in einer Menge von 1 bis 150 ppm, bezogen auf das Gesamtgewicht des Trägers und berechnet als Element.

Demnach beschreibt die vorliegende Erfindung auch ein wie oben beschriebenes Verfahren zur Herstellung eines Katalysators und einen Katalysator, herstellbar durch dieses Verfahren, wie oben beschrieben, wobei der Träger Magnesium in einer Menge von höchstens 800 ppm, und Calcium in einer Menge von 10 bis 1500 ppm, jeweils bezogen auf das Gesamtgewicht des Trägers und berechnet als Element, umfasst. Ebenso betrifft die vorliegende Erfindung auch einen wie oben beschriebenen Katalysator an sich, wobei der Träger Magnesium in einer Menge von höchstens 800 ppm, und Calcium in einer Menge von 10 bis 1500 ppm, jeweils bezogen auf das Gesamtgewicht des Trägers und berechnet als Element, umfasst.

Besonders bevorzugt umfasst der Träger Natrium in einer Menge von 10 bis 1500 ppm, Kalium in einer Menge von höchstens 1000 ppm, Magnesium in einer Menge von höchstens 800 ppm, und Calcium in einer Menge von 10 bis 1500 ppm, jeweils bezogen auf das Gesamtgewicht des Trägers und berechnet als Element.

Im Rahmen der Erfindung ist es besonders bevorzugt, dass der Träger Zirkon enthält. Enthält der Träger Zirkon, so enthält er Zirkon bevorzugt in einer Menge im Bereich von 1 bis 10000 ppm, weiter bevorzugt im Bereich von 10 bis 8000 ppm, weiter bevorzugt im Bereich von 50 bis 6000 ppm und besonders bevorzugt im Bereich von 50 bis 5000 ppm, berechnet als Metall und bezogen auf das Gesamtgewicht des Trägers.

Zusätzlich zu dem Zink, welches in Schritt (ii) auf den Träger aufgebracht wird, kann der gemäß (i) bereitgestellte Träger an sich, insbesondere der alpha-Aluminiumoxid-Träger, Zink als weiteren Bestandteil enthalten. Enthält der Träger als weiteren Bestandteil Zink, so enthält er dies in einer Menge von höchstens 1000 ppm, bevorzugt in einer Menge von höchstens 250 ppm, weiter bevorzugt in einer Menge im Bereich von 1 bis 250 ppm, berechnet als Element und bezogen auf das Gesamtgewicht des Trägers

Demnach betrifft die vorliegende Erfindung auch ein Verfahren, wie oben beschrieben, und einen Katalysator, herstellbar oder hergestellt durch dieses Verfahren, wobei der gemäß (i) bereitgestellte Trägers 1 bis 1000 ppm Zink und 1 bis 10000 ppm Zirkon, jeweils berechnet als Element und bezogen auf das Gesamtgewicht des Trägers, enthält.

Gemäß einer alternativen bevorzugten Ausführungsform enthält der gemäß (i) bereitgestellte Träger kein Zink.

Enthält der Träger weitere Bestandteile ausgewählt aus der Gruppe bestehend aus Gallium, Hafnium, Bor, Fluor, Kupfer, Nickel, Mangan, Eisen, Cer, Titan und Chrom, so enthält er diese bevorzugt jeweils in eine Menge von höchstens 500 ppm, jeweils berechnet als Metall und bezogen auf das Gesamtgewicht des Trägers.

Die erfindungsgemäß eingesetzten Träger enthalten bevorzugt eine BET-Oberfläche, bestimmt gemäß DIN ISO 9277, von 0,1 bis 5 m²/g, weiter bevorzugt im Bereich von 0,1 bis 2 m²/g, weiter bevorzugt im Bereich von 0,5 bis 1,5 m²/g, weiter bevorzugt im Bereich von 0,7 bis 1,3 m²/g, weiter bevorzugt im Bereich von 0,7 bis 1,2 m²/g und besonders bevorzugt im Bereich von 0,7 bis 1,0 m²/g.

Demnach betrifft die vorliegende Erfindung auch ein wie oben beschriebenes Verfahren zur Herstellung eines Katalysators und einen Katalysator, herstellbar durch dieses Verfahren, wobei der Träger eine BET-Oberfläche, bestimmt gemäß DIN ISO 9277, im Bereich von 0,1 bis 5 m²/g, weiter bevorzugt im Bereich von 0,1 bis 2 m²/g, weiter bevorzugt im Bereich von 0,5 bis 1,5 m²/g, weiter bevorzugt im Bereich von 0,7 bis 1,3 m²/g, weiter bevorzugt im Bereich von 0,7 bis 1,3 m²/g, weiter bevorzugt im Bereich von 0,7 bis 1,2 m²/g und besonders bevorzugt im Bereich von 0,7 bis 1,0 m²/ aufweist. Ebenso betrifft die vorliegende Erfindung auch einen wie oben beschriebenen Katalysator an sich, wobei der Träger eine BET-Oberfläche, bestimmt gemäß DIN ISO 9277, im Bereich von 0,1 bis 5 m²/g, weiter bevorzugt im Bereich von 0,1 bis 2 m²/g, weiter bevorzugt im Bereich von 0,5 bis 1,5 m²/g, weiter bevorzugt im Bereich von 0,7 bis 1,3 m²/g, weiter bevorzugt im Bereich von 0,7 bis 1,3 m²/g, weiter bevorzugt im Bereich von 0,7 bis 1,2 m²/g und besonders bevorzugt im Bereich von 0,7 bis 1,0 m²/g aufweist.

Weiter weisen die erfindungsgemäßen Träger bevorzugt Poren mit Durchmessern im Bereich von 0,1 bis 100 µm auf, wobei die Porenverteilung monomodal oder polymodal, beispielsweise bimodal, trimodal oder tetramodal sein kann. Vorzugsweise weisen die Träger eine bimodale Porenverteilung auf. Weiter bevorzugt weisen die Träger eine bimodale Porenverteilung mit Peakmaxima im Bereich von 0,1 bis 10 µm und 15 bis 100 um, bevorzugt im Bereich von 0,1 bis 5 µm und 17 bis 80 µm, weiter bevorzugt im Bereich von 0,1 bis 3 µm und 20 bis 50 µm, weiter bevorzugt im Bereich von 0,1 bis 1,5 µm und 20 bis 40 µm auf. Die Porendurchmesser werden durch Hg-Porosimetrie (DIN 66133) bestimmt. Der Begriff "bimodale Porenverteilung mit Peakmaxima im Bereich von 0,1 bis 10 µm und 15 bis 100 µm", wie oben verwendet, besagt, dass eines der beiden Peakmaxima im Bereich von 0,1 bis 10 µm liegt und das andere Peakmaximum im Bereich von 15 bis 100 µm liegt.

Demnach betrifft die vorliegende Erfindung auch ein wie oben beschriebenes Verfahren zur Herstellung eines Katalysators und einen Katalysator, herstellbar durch dieses Verfahren, wobei der Träger eine polymodale Porenverteilung aufweist, bevorzugt eine bimodale Porenverteilung, weiter bevorzugt eine bimodale Porenverteilung mindestens enthaltend Poren mit Porendurchmessern im Bereich von 0,1 bis 10 µm und Poren mit Porendurchmessern im Bereich von 15 bis 100 µm, bestimmt durch Hg-Porosimetrie gemäß DIN 66133. Ebenso betrifft die vorliegende Erfindung auch einen wie oben beschriebenen Katalysator an sich, wobei der Träger eine polymodale Porenverteilung aufweist, bevorzugt eine bimodale Porenverteilung, weiter bevorzugt eine bimodale Porenverteilung, mindestens enthaltend Poren mit Porendurchmessern im Bereich von 0,1 bis 10 µm und Poren mit Porendurchmessern im Bereich von 15 bis 100 µm, bestimmt durch Hg-Porosimetrie gemäß DIN 66133.

Die geometrische Form der Träger ist im Allgemeinen von untergeordneter Bedeutung, zweckmäßigerweise sollten die Träger aber in Form von Partikeln vorliegen, die eine ungehinderte Diffusion der Reaktionsgase an einen möglichst großen Teil der mit der katalytisch aktiven, mit Silberpartikeln belegten und gegebenenfalls mit weiteren Promotoren belegten äußeren und inneren Oberfläche des Trägers ermöglichen. Außerdem muss durch die gewählte geometrische Form des Trägers ein möglichst geringer Druckverlust über die gesamte Reaktorlänge gewährleistet werden.

Gemäß einer bevorzugten Ausführungsform wird der Träger als Formkörper, beispielsweise als Strang, Hohlstrang, Sternstrang, Kugel, Ring oder Hohlring eingesetzt. Bevorzugt wird der Katalysator in Form eines Hohlrings eingesetzt. Üblicherweise haben die Formkörper einen Volumenäquivalentdurchmesser von etwa 2,5 bis 12 mm, bevorzugt von etwa 3 bis 10 mm. Der Begriff "Volumenäquivalentdurchmesser" gibt den Durchmesser einer Kugel mit gleichem Volumen an wie der betrachtete Formkörper. Bevorzugt ist der Träger ein Formkörper mit der Geometrie eines Hohlkörpers. Insbesondere bevorzugt sind Zylinder mit folgenden Geometrien (Außendurchmesser x Länge x Innendurchmesser, jeweils angegeben in mm): 5x5x2, 6x6x3, 7x7x3, 8x8x3, 8x8,5x3, 8x8,5x3,5, 8,5x8x3,5, 8,5x8x3, 9x9x3, 9,5x9x3, 9,5x9x3,5. Jede Längenangabe umfasst Toleranzen im Bereich von ± 0,5 mm.

Die Wasserabsorption der Träger liegt bevorzugt im Bereich von 0,35 ml/g bis 0,65 ml/g, bevorzugt im Bereich von 0,42 ml/g bis 0,52 ml/g, bestimmt durch eine Vakuumkaltwasseraufnahme.

Im Allgemeinen kann ein geeigneter Katalysatorträger für die vorliegende Erfindung durch Mischen des Aluminiumoxids mit Wasser oder einer anderen geeigneten Flüssigkeit, mit einem Ausbrennmaterial oder einem Porenbildner und mindestens einem Bindemittel hergestellt werden. Geeignete Porenbildner sind beispielsweise Cellulose und Cellulosederivate, wie z.B. Methylcellulose, Ethylcellulose, Carboxymethylcellulose, oder Polyolefine, wie Polyethylene und Polypropylene, oder natürliche Ausbrennmaterialien wie z.B. Walnussschalenmehl.

Die Porenbildner werden so gewählt, dass sie bei den gewählten Ofentemperaturen der Calcination vom Aluminiumoxid zum fertigen alpha-Aluminiumoxidträger vollständig ausgebrannt werden. Es ist wichtig, dass sie vollständig ausgebrannt werden, da dadurch die Porosität und die Form der Poren entscheidend beeinflusst werden. Geeignete Bindemittel bzw. Verstrangungs- und Extrusionshilfsmittel sind beispielsweise in EP 0 496 386 B2 beschrieben. Zu nennen sind beispielhaft Aluminiumoxid-Gele mit Salpetersäure oder Essigsäure, Cellulose, z.B. Methyl-, Ethylcellulose, oder Carboxyethylcellulose, oder Methyl- oder Ethylstearat, Polyolefinoxide, Wachse und ähnliche.

Die durch Mischen gebildete Paste kann durch Extrusion in die gewünschte Form gebracht werden. Zur Unterstützung des Extrusionsprozesses können Extrusionshilfsmittel verwendet werden.

Üblicherweise wird der wie oben beschrieben erhaltene Formkörper im Anschluss an das Verformen gegebenenfalls getrocknet und unter Erhalt des Aluminiumoxidträgers gemäß (i) calciniert. Das Calcinieren erfolgt üblicherweise bei Temperaturen im Bereich von 1200 °C bis 1600 °C. Es ist gängig, den Aluminiumoxidträger nach dem Calcinieren geeignet zu waschen, um lösliche Bestandteile zu entfernen.

Geeignete Träger sind beispielsweise erhältlich bei NorPro Co.

### Schritt (ii)

In Schritt (ii) wird Zink auf dem Träger gemäß (i) in einer Menge von 10 ppm bis 1500 ppm, jeweils berechnet als Element und bezogen auf das Gesamtgewicht des oben beschriebenen Trägers, durch Inkontaktbringen des Trägers mit mindestens einem Gemisch G1 umfassend mindestens eine Zinkverbindung aufgebracht.

Vorzugsweise enthält das Gemisch G1 die mindestens eine Zinkverbindung gelöst in mindestens einem Lösungsmittel, insbesondere gelöst in Wasser. Die mindestens eine Zinkverbindung ist dabei vorzugsweise ausgewählt aus der Gruppe bestehend aus Zinkchlorid, Zinknitrat, Zinkacetat, Zinkbromid, Zinkiodid, Zinkcarbonat, Zinkhydroxid, Zinkoxalat und Zinkacetylacetonat. Das Gemisch G1 kann weiterhin ein Komplexierungsmittel, wie Ethanolamin, EDTA, 1,3- oder 1,2-Propandiamin, Ethylendiamin und/oder Alkalioxalat enthalten.

Insbesondere enthält G1 kein Silber und/oder keine Silberverbindung.

Gemäß einer besonders bevorzugten Ausführungsform der Erfindung enthält das Gemisch G1 Zinkacetat, Ethylendiamin und bevorzugt mindestens ein Lösungsmittel, wobei das Lösungsmittel weiter bevorzugt Wasser ist.

Demnach betrifft die vorliegende Erfindung auch ein Verfahren zur Herstellung eines Katalysators umfassend Silber und Zink, aufgebracht auf einem Träger, und einen Katalysator, hergestellt oder herstellbar durch dieses Verfahren, wobei das Verfahren mindestens umfasst
(i) Bereitstellen eines Trägers,
(ii) Aufbringen von Zink auf dem Träger gemäß (i) in einer Menge von 10 ppm bis 1500 ppm, jeweils berechnet als Element und bezogen auf das Gesamtgewicht des Trägers gemäß (i), durch Inkontaktbringen des Trägers mit mindestens einem Gemisch G1 umfassend Zinkacetat und Ethylendiamin,
(iii) Trocknen und/oder Calcinieren des gemäß (ii) erhaltenen Trägers,
(iv) Aufbringen von Silber auf dem getrockneten und/oder calcinierten Träger durch Inkontaktbringen des Trägers mit mindestens einem Gemisch G2 umfassend mindestens eine Silberverbindung.

Das Aufbringen kann prinzipiell durch jedes geeignete Verfahren erfolgen, beispielsweise durch Tränken des Trägers und optional anschließendes Trocknen. Besonders bevorzugt erfolgt das Aufbringen durch Vakuumimprägnierung bei Raumtemperatur. Bei der Vakuumimprägnierung wird der Träger bevorzugt zunächst bei einem Druck im Bereich von höchstens 500 mbar, weiter bevorzugt bei einem Druck von höchstens 250 mbar und besonders bevorzugt bei einem Druck von höchsten 30 mbar behandelt (Vakuumbehandlung). Besonders bevorzugt erfolgt dies bei einer Temperatur im Bereich von 1°C bis 80°C, weiter bevorzugt bei einer Temperatur im Bereich von 3°C bis 50°C, weiter bevorzugt bei einer Temperatur im Bereich von 5°C bis 30°C, und besonders bevorzugt bei Raumtemperatur. Die Vakuumbehandlung erfolgt dabei vorzugsweise für eine Zeit von mindestens 1 min, bevorzugt von mindestens 5 min, weiter bevorzugt für eine Zeit im Bereich von 5 min bis 120 min, insbesondere im Bereich von 10 min bis 45 min, besonders bevorzugt im Bereich von 15 min bis 30 min.

Im Anschluss an die Vakuumbehandlung wird Gemisch G1 mit dem Träger in Kontakt gebracht. Bevorzugt ist G1 eine Lösung, wobei die Lösung im Vakuum aufgetropft oder aufgesprüht, bevorzugt aufgesprüht, wird. Das Aufbringen erfolgt dabei bevorzugt mittels einer Düse. Die Imprägnation erfolgt bei einem Druck im Bereich von höchstens 500 mbar, weiter bevorzugt bei einem Druck von höchstens 250 mbar und besonders bevorzugt bei einem Druck von höchsten 30 mbar. Während der Imprägnation wird der im Vakuum vorbehandelte Träger bevorzugt durch zum Beispiel eine Drehung des Imprägnationsgefäßes in Bewegung gehalten, um eine möglichst gleichmäßige Verteilung des Gemisches G1 auf dem Träger zu gewährleisten.

Was die Menge an Zink betrifft, die in (ii) auf den Träger aufgebracht wird, so wird, wie oben erwähnt, Zink bevorzugt in einer Menge von 10 bis 1500 ppm, weiter bevorzugt in einer Menge von 50 bis 1250 ppm und besonders bevorzugt in einer Menge von 150 bis 1100 ppm, berechnet als Element und bezogen auf das Gesamtgewicht des Trägers in (ii), auf den Träger aufgebracht.

Das Aufbringen gemäß (ii) kann dabei auch in mehr als einem Schritt, beispielsweise in 2, 3 oder 4 Schritten erfolgen. Zwischen den einzelnen Schritten kann der Träger jeweils optional getrocknet und/oder calciniert werden. Erfolgt das Aufbringen gemäß (ii) in mehr als einem Schritt, so ist die Gesamtmenge des nach allen Schritten auf dem Träger aufgebrachten Zinks ebenfalls im Bereich von 10 ppm bis 1500 ppm, wie oben beschrieben.

### Schritt (iii)

Nach dem Aufbringen wird der Träger getrocknet und/oder calciniert.

Das Trocknen kann dabei prinzipiell durch jede dem Fachmann bekannte Art erfolgen.

Im Rahmen der Erfindung erfolgt das Trocknen bevorzugt bei Temperaturen im Bereich von 2 bis 200 °C. Insbesondere erfolgt das Trocknen durch Vakuumbehandlung. Der Begriff "Vakuumbehandlung" bedeutet in diesem Zusammenhang, dass der Träger bei einem Druck im Bereich von höchstens 500 mbar, weiter bevorzugt bei einem Druck von höchstens 250 mbar und besonders bevorzugt bei einem Druck von höchstens 30 mbar, evakuiert wird. Bevorzugt erfolgt die Vakuumbehandlung bei einer Temperatur im Bereich von 2°C bis 50°C, weiter bevorzugt bei einer Temperatur im Bereich von 5°C bis 30°C, und besonders bevorzugt bei Raumtemperatur. Die Vakuumbehandlung kann dabei bei einer konstanten Temperatur erfolgen. Weiterhin sind Ausführungsformen umfasst, bei denen die Temperatur während der Vakuumbehandlung kontinuierlich oder diskontinuierlich geändert wird.

Die Vakuumbehandlung erfolgt dabei für eine Zeit von mindestens 1 min, bevorzugt von mindestens 5 min, weiter bevorzugt für eine Zeit im Bereich von 5 min bis 120 min, insbesondere im Bereich von 10 min bis 45 min, besonders bevorzugt im Bereich von 10 min bis 20 min.

Wird der Träger in Schritt (iii) calciniert, so erfolgt dieses Calcinieren bevorzugt bei Temperaturen im Bereich von 150 bis 1500 °C, bevorzugt im Bereich von 200 bis 1250 °C, und besonders bevorzugt im Bereich von 220 bis 1100 °C. Die Calcinierung kann dabei in einer Stufe oder auch in mindestens zwei Stufen erfolgen. Calcinieren in mehreren Stufen bedeutet in diesem Zusammenhang, dass der Katalysator zwischen den jeweiligen Calcinierungsstufen auf eine Temperatur im Bereich von 0 bis 50 °C, bevorzugt auf Raumtemperatur, abgekühlt wird. Die Temperaturen der einzelnen Calcinierungsstufen können dabei gleich sein oder sich voneinander unterscheiden. Bevorzugt unterscheiden sich die Temperaturen der einzelnen Calcinierungsstufen um mindestens 700° C. Werden beispielsweise zwei Calcinierungsschritte in (iii) durchgeführt so erfolgt der erste Calcinierungsschritt bevorzugt bei einer Temperatur im Bereich von 150° bis 400 °C, weiter bevorzugt bei einer Temperatur im Bereich von 200 bis 350 °C, und besonders bevorzugt im Bereich von 220 bis 300 °C. Der zweite Calcinierungsschritt erfolgt bevorzugt bei einer Temperatur im Bereich von 700° bis 1500 °C, bevorzugt im Bereich von 800 bis 1200 °C, und besonders bevorzugt im Bereich von 900 bis 1100 °C.

Die Calcinierungsdauer der mindestens einen Calcinierungsstufe liegt dabei im Allgemeinen bei mindestens 5 Minuten oder mehr, beispielsweise im Bereich von 5 Minuten bis 24 Stunden oder im Bereich von 10 Minuten bis 12 Stunden, wobei die Calcinierungsdauem der jeweiligen Stufen, im Falle, dass mehrere Calcinierungsstufen durchgeführt werden, gleich sein können oder sich voneinander unterscheiden können.

Die Calcinierung kann dabei in jeder Stufe unabhängig voneinander bei einer konstanten Temperatur erfolgen; weiterhin sind Ausführungsformen umfasst, bei denen die Temperatur während der Calcinierungsdauer kontinuierlich oder diskontinuierlich geändert wird.

Bevorzugt wird der Träger in Schritt (iii) calciniert, wobei die Calcinierung besonders bevorzugt in zwei Calcinierungsstufen erfolgt.

Die Calcinierung kann unter jeder dafür geeigneten Gasatmosphäre erfolgen, beispielsweise in einem Inertgas oder einer Mischung aus einem Inertgas und bis zu 21 Vol.-% Sauerstoff. Als Inertgas sei beispielsweise Stickstoff, Argon, Helium, Wasserdampf, Kohlendioxid und eine Kombination der mindestens zwei der vorstehend genannten Inertgase genannt. Wird die Calcinierung in einem Inertgas durchgeführt, so wird Stickstoff besonders bevorzugt. Gemäß einer alternativen bevorzugten Ausführungsform wird Luft und/oder Magerluft verwendet. Erfolgt die Calcinierung in mindestens zwei Calcinierungsstufen, bevorzugt in zwei Calcinierungsstufen, so kann die Calcinierung der einzelnen Stufen in verschiedenen Gasatmosphären erfolgen. Bevorzugt erfolgt die Calcinierung in beiden Stufen unter Luft und/oder Magerluft.

Der Begriff Magerluft, wie er im Rahmen der vorliegenden Erfindung verwendet wird, bedeutet Luft mit einem Sauerstoffgehalt im Bereich von 0,1 bis 19 Vol.-% und einem Stickstoffgehalt von 81 bis 99,9 Vol.-%.

Weiter wird die Calcinierung bevorzugt in einem Muffelofen, in einem Drehofen, Umluftofen und/oder einem Band-Calcinierofen durchgeführt. Wird die Calcinierung in mindestens zwei Stufen, bevorzugt in zwei Stufen durchgeführt, so erfolgt die Calcinierung bevorzugt in verschiedenen Öfen, beispielsweise wird zunächst in einem Umluftofen bzw. Bandcalcinierofen calciniert und der zweite Calcinierungsschritt erfolgt in einem Muffelofen.

Besonders bevorzugt wird der Träger in Schritt (iii) zunächst bei einer Temperatur im Bereich von 220 bis 350 °C für eine Zeit im Bereich von 5 min bis 2 h, bevorzugt im Bereich von 5 min bis 1 h, ganz besonders im Bereich von 10 min bis 20 min, bevorzugt unter Luft, calciniert, wobei diese Calcinierung besonders bevorzugt im Umluftofen durchgeführt wird. Gemäß eine weiter bevorzugten Ausführungsform, wird der so erhaltene, calcinierte Träger in Schritt (iii) in einem zweiten Calcinierungsschritt bei einer Temperatur im Bereich von 900 bis 1100 °C, für eine Zeit im Bereich von 10 min bis 12 h, bevorzugt im Bereich von 2 h bis 11 h, ganz besonders im Bereich von 5 h bis 10 h, bevorzugt unter Luft, calciniert, wobei diese Calcinierung besonders bevorzugt im Muffelofen durchgeführt wird.

Demnach betrifft die vorliegende Erfindung auch ein Verfahren zur Herstellung eines Katalysators umfassend Silber und Zink, aufgebracht auf einem Träger, und einen Katalysator, hergestellt oder herstellbar durch dieses Verfahren, wobei das Verfahren mindestens umfasst
(i) Bereitstellen eines Trägers,
(ii) Aufbringen von Zink auf dem Träger gemäß (i) in einer Menge von 10 ppm bis 1500 ppm, jeweils berechnet als Element und bezogen auf das Gesamtgewicht des Trägers gemäß (i), durch Inkontaktbringen des Trägers mit mindestens einem Gemisch G1 umfassend mindestens eine Zinkverbindung,
(iii) gegebenenfalls Trocknen des gemäß (ii) erhaltenen Trägers und Calcinieren des gemäß (ii) erhaltenen gegebenenfalls getrockneten Trägers, wobei das Calcinieren umfasst
   (aa) Calcinieren des gegebenenfalls getrockneten Trägers bei einer Temperatur im Bereich von 220 bis 350 °C, bevorzugt für eine Zeit im Bereich von 5 min bis 2 h, wobei das Calcinieren bevorzugt unter Luft durchgeführt wird und wobei diese Calcinierung besonders bevorzugt im Umluftofen durchgeführt wird
   (bb) Calcinieren des gemäß (aa) erhaltenen Trägers in einem Calcinierungsschritt bei einer Temperatur im Bereich von 900 bis 1100 °C, bevorzugt für eine Zeit im Bereich von 10 min bis 12 h, wobei das Calcinieren bevorzugt unter Luft durchgeführt wird und wobei diese Calcinierung besonders bevorzugt im Muffelofen durchgeführt wird,
(iv) Aufbringen von Silber auf dem gegebenenfalls getrockneten, calcinierten Träger durch Inkontaktbringen des Trägers mit mindestens einem Gemisch G2 umfassend mindestens eine Silberverbindung.

### Schritt (iv)

Das Aufbringen von Silber auf dem gemäß (iii) erhaltenen Träger erfolgt, wie oben beschrieben, durch Inkontaktbringen des Trägers mit mindestens einem Gemisch G2 umfassend mindestens eine Silberverbindung.

Was das Aufbringen von Silber auf dem Träger betrifft, so eignen sich generell alle Verfahren, bei denen Silber in geeigneter Weise auf dem Träger aufgebracht wird. Bevorzugt wird das mindestens eine Gemisch G2, welches mindestens eine Silberverbindung umfasst, durch Tränk- oder Sprüh- oder Mischverfahren auf dem Träger, aufgebracht. Beispielhaft seien die Herstellungsverfahren für Silberkatalysatoren genannt, wie sie in DE-A 2300512, DE-A 2521906, EP-A 14457, EP-A 85237, EP-A 384312, DE-A 2454972, DE-A 3321895, EP-A 229465, DE-A 3150205, EP-A 172565 und EP-A 357293 offenbart sind.

Besonders bevorzugt erfolgt das Aufbringen von Silber durch Vakuumimprägnation bei Raumtemperatur. Bei der Vakuumimprägnierung wird der Träger, wie oben beschrieben, bevorzugt zunächst bei einem Druck im Bereich von höchstens 500 mbar, weiter bevorzugt bei einem Druck von höchstens 250 mbar und besonders bevorzugt bei einem Druck von höchsten 30 mbar behandelt. Besonders bevorzugt erfolgt dies bei einer Temperatur im Bereich von 1°C bis 80°C, weiter bevorzugt bei einer Temperatur im Bereich von 3°C bis 50°C, weiter bevorzugt bei einer Temperatur im Bereich von 5°C bis 30°C, und besonders bevorzugt bei Raumtemperatur. Die Vakuumbehandlung erfolgt dabei vorzugsweise für eine Zeit von mindestens 1 min, bevorzugt von mindestens 5 min, weiter bevorzugt für eine Zeit im Bereich von 5 min bis 120 min, insbesondere im Bereich von 10 min bis 45 min, besonders bevorzugt im Bereich von 15 min bis 30 min.

Im Anschluss an die Vakuumbehandlung wird mindestens Gemisch G2 mit dem Träger in Kontakt gebracht. Bevorzugt wird Gemisch G2 aufgetropft oder aufgesprüht, bevorzugt aufgesprüht. Das Aufbringen erfolgt dabei bevorzugt mittels einer Düse. Die Imprägnation erfolgt bei einem Druck im Bereich von höchstens 500 mbar, weiter bevorzugt bei einem Druck von höchstens 250 mbar und besonders bevorzugt bei einem Druck von höchstens 30 mbar. Während der Imprägnation wird der im Vakuum vorbehandelte Träger bevorzugt im Imprägnationsgefäß in Bewegung gehalten, beispielsweise durch Drehung, um eine möglichst gleichmäßige Verteilung des Gemisches G2 auf dem Träger zu gewährleisten.

Gemisch G2 enthält Silber bevorzugt in Form mindestens einer Silberverbindung. Bevorzugt wird die Silberverbindung gelöst, insbesondere gelöst in Wasser, aufgebracht. Demnach enthält G2 weiterhin bevorzugt mindestens ein Lösungsmittel, bevorzugt Wasser. Um die Silberverbindung in löslicher Form zu erhalten, kann der Silberverbindung, wie beispielsweise Silber(I)-oxid oder Silber(I)-oxalat, ein Komplexierungsmittel, wie Ethanolamin, EDTA, 1,3- bzw. 1,2-Propandiamin, Ethylendiamin und/oder Alkalioxalat zugesetzt werden, das gleichzeitig auch als Reduktionsmittel wirken kann. Besonders bevorzugt wird Silber in Form einer Silberaminverbindung, insbesondere in Form einer Silberethylendiaminverbindung, aufgebracht.

Demnach enthält G2 gemäß einer bevorzugten Ausführungsform mindestens ein Komplexierungsmittel, insbesondere Ethanolamin, EDTA, 1,3- oder 1,2-Propandiamin, und/oder Ethylendiamin.

Enthält G2 mindestens ein Komplexierungsmittel, so enthält G2 mindestens einen Teil des Silbers in Form einer Silberkomplexverbindung. Besonders bevorzugt enthält G2 mindestens einen Teil des Silbers als Silberethylendiaminverbindung. Besonders bevorzugt umfasst G2 Wasser, Silberethylendiamin, Oxalat und optional Ethylendiamin.

Demnach betrifft die vorliegende Erfindung auch ein Verfahren, wie oben beschrieben, wobei G2 Wasser und Silberethylendiamin umfasst. Ebenso betrifft die vorliegende Erfindung einen Katalysator, herstellbar oder hergestellt durch dieses Verfahren.

Insbesondere enthält G2 weder metallisches Zink noch eine Zinkverbindung.

Was die Menge an Silber betrifft, die in (iv) auf den Träger aufgebracht wird, so wird wie oben erwähnt, Silber bevorzugt in einer Menge von 1 bis 50 Gew.-%, weiter bevorzugt in einer Menge von 5 bis 35 Gew.-%, und besonders bevorzugt in einer Menge von 10 bis 25 Gew.-%, berechnet als Element und bezogen auf das Gesamtgewicht des Katalysators, auf den Träger aufgebracht.

Das Aufbringen in (iv) kann dabei auch in mehr als einem Schritt, beispielsweise in 2, 3 oder 4 Schritten erfolgen. Zwischen den einzelnen Schritten kann der Träger jeweils optional getrocknet und/oder calciniert werden. Erfolgt das Aufbringen gemäß (iv) in mehr als einem Schritt, so ist die Gesamtmenge des nach allen Schritten auf dem Träger aufgebrachten Silbers ebenfalls im Bereich von 1 bis 50 Gew.-%, weiter bevorzugt im Bereich von 5 bis 35 Gew.-%, und besonders bevorzugt in einer Menge von 10 bis 25 Gew.-%, berechnet als Element und bezogen auf das Gesamtgewicht des Katalysators, wie oben beschrieben.

Nach dem Aufbringen des Silbers können sich mindestens ein Nachbehandlungsschritt, beispielsweise ein, zwei oder mehr Trocknungsschritte anschließen. Das Trocknen erfolgt dabei üblicherweise bei Temperaturen im Bereich von 2 bis 200 °C. Bevorzugt handelt es sich bei dem Nachbehandlungsschritt um eine Trocknung mittels Vakuumbehandlung, wie oben beschrieben. Bevorzugt erfolgt diese Evakuierung bei einem Druck im Bereich von höchstens 500 mbar, weiter bevorzugt bei einem Druck von höchstens 250 mbar und besonders bevorzugt bei einem Druck von höchsten 30 mbar. Bevorzugt erfolgt die Vakuumbehandlung bei einer Temperatur im Bereich von 2°C bis 50°C, weiter bevorzugt bei einer Temperatur im Bereich von 5°C bis 30°C, und besonders bevorzugt bei Raumtemperatur. Die Vakuumbehandlung erfolgt dabei für eine Zeit von mindestens 1 min, bevorzugt von mindestens 5 min, weiter bevorzugt für eine Zeit im Bereich von 5 min bis 120 min, insbesondere im Bereich von 10 min bis 45 min, besonders bevorzugt im Bereich von 10 min bis 20 min.

Nach Aufbringen des Silbers und gegebenenfalls nach dem mindestens einen Trocknungsschritt schließt sich bevorzugt mindestens ein Calcinierungsschritt an.

Demnach betrifft die vorliegende Erfindung auch ein Verfahren, wie oben beschrieben, und einen Katalysator, herstellbar oder hergestellt nach diesem Verfahren, wobei das Verfahren weiterhin umfasst
(v) Trocknen und/oder Calcinieren des Trägers gemäß (iv).

Wird in Schritt (v) calciniert, so erfolgt dieses Calcinieren bevorzugt bei Temperaturen im Bereich von 150 bis 750 °C, bevorzugt im Bereich von 200 bis 500 °C, und besonders bevorzugt im Bereich von 220 bis 350 °C, wobei die Calcinierungsdauer im Allgemeinen bei mindestens 5 Minuten oder mehr, beispielsweise im Bereich von 5 Minuten bis 24 Stunden oder im Bereich von 10 Minuten bis 12 Stunden liegt.

Besonders bevorzugt liegt die Calcinierungsdauer im Bereich von 5 Minuten bis 3 Stunden. Die Calcinierung kann dabei bei einer konstanten Temperatur erfolgen; weiterhin sind Ausführungsformen umfasst, bei denen die Temperatur während der Calcinierungsdauer kontinuierlich oder diskontinuierlich geändert wird.

Die Calcinierung kann, wie oben beschrieben, unter jeder dafür geeigneten Gasatmosphäre erfolgen, beispielswiese in einem Inertgas oder einer Mischung aus einem Inertgas und bis zu 21 Vol.-% Sauerstoff. Als Inertgas seien beispielsweise Stickstoff, Argon, Helium, Kohlendioxid, Wasserdampf und eine Kombination aus mindestens zwei der vorstehend genannten Inertgase genannt. Wird die Calcinierung in einem Inertgas durchgeführt, so wird Stickstoff besonders bevorzugt. Gemäß einer alternativen bevorzugten Ausführungsform wird Luft und/oder Magerluft verwendet. Weiter wird die Calcinierung bevorzugt in einem Muffelofen, in einem Drehofen, Umluftofen und/oder einem Band-Calcinierofen durchgeführt.

### Promotoren

Bevorzugt enthält der Katalysator neben Silber und Zink mindestens einen Promotor. Dieser mindestens eine Promotor wird bevorzugt durch Tränk- oder Sprüh- oder Mischverfahren auf dem Träger aufgebracht.

Was den Zeitpunkt des Aufbringens des mindestens einen Promotors betrifft, so kann dieser im Anschluss an das oben beschriebene Calcinieren gemäß Schritt (v) durchgeführt werden. Alternativ ist es möglich, den mindestens einen Promotor zusammen mit der Silberverbindung auf dem Träger aufzubringen.

Demnach sind im Rahmen der Erfindung Ausführungsformen umfasst, bei denen der mindestens eine Promotor, also beispielsweise fünf verschiedene Promotoren, vier verschiedene Promotoren, drei verschiedene Promotoren, zwei verschiedene Promotoren oder ein Promotor auf dem Träger aufgebracht werden und der so behandelte Träger erst anschließend, wie oben beschrieben, unter Erhalt eines erfindungsgemäßen Katalysators calciniert wird.

Ebenso sind Ausführungsformen umfasst, bei denen der Promotor in Schritt (ii), vor Schritt (ii) oder nach Schritt (ii) und/oder (iii) aufgebracht wird. Ebenso ist es möglich, den mindestens einen Promotor nach Schritt (iv) aufzubringen.

Weiterhin sind Ausführungsformen umfasst, bei denen ein Teil des mindestens einen Promotors, der erst nach Aufbringen des Silbers gemäß (iv) auf den Träger aufgebracht wird, und der verbleibende Teil mit Silber in (iv) gleichzeitig aufgebracht wird.

Im Rahmen der Erfindung wird der mindestens eine Promotor bevorzugt in Schritt (iv) gleichzeitig mit Silber aufgebracht. Dabei kann der mindestens eine Promotor in einer weiteren Lösung G2' auf den Träger aufgebracht werden. Bevorzugt wird der Promotor zusammen mit Silber aufgebracht. In diesem Fall, umfasst G2 zusätzlich den mindestens einen Promotor.

Enthält der Katalysator mindestens einen Promotor, so wird dieser mindestens eine Promotor im erfindungsgemäßen Verfahren zur Herstellung des Katalysators bevorzugt in Form von Verbindungen auf den Träger aufgebracht, beispielsweise in Form von Komplexen oder in Form von Salzen, beispielsweise in Form von Halogeniden, beispielsweise in Form von Fluoriden, Bromiden oder Chloriden, oder in Form von Carboxylaten, Nitraten, Sulfaten oder Sulfiden, Phosphaten, Cyaniden, Hydroxiden, Carbonaten oder als Salze von Heteropolysäuren.

### Rhenium als Promotor

Besonders bevorzugt enthält der Katalysator Rhenium als Promotor. Bevorzugt wird Rhenium zusammen mit Silber in Schritt (iv) des erfindungsgemäßen Verfahrens aufgebracht. Das Rhenium wird insbesondere als Verbindung, beispielsweise als Halogenid, Oxyhalogenid, Oxid oder als Säure aufgebracht. Weiterhin kann Rhenium in Form von Salzen der Heteropolysäuren des Rheniums, beispielsweise als Rhenat oder Perrhenat, im erfindungsgemäßen Herstellungsverfahren eingesetzt werden.

Demnach betrifft die vorliegende Erfindung auch ein Verfahren, wie oben beschrieben, und einen Katalysator, herstellbar oder hergestellt nach diesem Verfahren, wobei der Katalysator Rhenium als Promotor enthält, und wobei das Rhenium in Schritt (iv) auf dem getrockneten und/oder calcinierten Träger gemäß (iii) durch Imprägnieren mit dem Gemisch G2, welches zusätzlich mindestens eine Rheniumverbindung umfasst, aufgebracht wird.

Sollte Rhenium als Promotor eingesetzt werden, wird es bevorzugt als eine Verbindung, ausgewählt aus der Gruppe bestehend aus Ammoniumperrhenat, Rhenium(III)-chlorid, Rhenium(V)-chlorid, Rhenium(V)-fluorid, Rhenium(VI)-oxid und Rhenium(VII)-oxid eingesetzt. Besonders bevorzugt wird im Rahmen der Erfindung Rhenium als Ammoniumperrhenat auf den Träger aufgebracht.

Was die Menge an Rhenium betrifft, so enthält der Katalysator Rhenium bevorzugt in einer Menge von 50 ppm bis 1000 ppm, weiter bevorzugt in einer Menge von 100 ppm bis 800 ppm, weiter bevorzugt in einer Menge von 200 ppm bis 600 ppm, und besonders bevorzugt in einer Menge von 250 ppm bis 450 ppm, bezogen auf das Gesamtgewicht des Katalysators und berechnet als Element.

Demnach betrifft die vorliegende Erfindung auch ein Verfahren, wie oben beschrieben, und einen Katalysator, herstellbar oder hergestellt nach diesem Verfahren, wobei der Katalysator Rhenium als Promotor umfasst, bevorzugt in einer Menge von 50 ppm bis 1000 ppm, bezogen auf das Gesamtgewicht des Katalysators und berechnet als Element. Ebenso beschreibt die vorliegende Erfindung einen Katalysator an sich, wie oben beschrieben, wobei der Katalysator Rhenium als Promotor umfasst, bevorzugt in einer Menge von 50 ppm bis 1000 ppm, bezogen auf das Gesamtgewicht des Katalysators und berechnet als Element.

### Weitere Promotoren

Gemäß einer besonders bevorzugten Ausführungsform enthält der Katalysator zusätzlich mindestens einen weiteren Promotor. Insbesondere ist dieser weitere Promotor ausgewählt aus Elementen der Gruppe IA, VIB, VIIB und VIA des Periodensystems der Elemente, besonders bevorzugt ausgewählt aus der Gruppe bestehend aus Wolfram, Lithium, Schwefel, Cäsium, Chrom, Mangan, Molybdän und Kalium.

Demnach betrifft die vorliegende Erfindung auch ein Verfahren, wie oben beschrieben, und einen Katalysator, herstellbar oder hergestellt nach diesem Verfahren, wobei der Katalysator zusätzlich mindestens einen weiteren Promotor ausgewählt aus Elementen der Gruppe IA, VIB, VIIB und VIA des Periodensystems der Elemente, bevorzugt ausgewählt aus der Gruppe bestehend aus Wolfram, Lithium, Schwefel, Cäsium, Chrom, Mangan, Molybdän und Kalium, enthält.

Gemäß einer besonders bevorzugten Ausführungsform enthält der Katalysator zusätzlich Cäsium, Lithium, Wolfram und Schwefel als Promotoren.

Enthält der Katalysator mindestens einen weiteren Promotor, so enthält er bevorzugt eine Gesamtmenge dieser weiteren Promotoren in einer Menge von 10 bis 2000 ppm, bevorzugt in einer Menge von 10 bis 1700 ppm, weiter bevorzugt jeweils in einer Menge von 50 bis 1500 ppm und besonders bevorzugt jeweils in einer Menge von 80 bis 1200 ppm, bezogen auf das Gesamtgewicht des Katalysators und berechnet als Summe der Elemente.

Enthält der Katalysator Wolfram als Promotor, wie oben beschrieben, so wird das Wolfram bevorzugt als Wolframverbindung auf den Träger aufgebracht. Hier ist im Prinzip jegliche geeignete Wolframverbindung verwendbar. Beispielsweise wird Wolfram in Form von Wolframat oder Wolframsäure aufgebracht.

Enthält der Katalysator Lithium als Promotor, wie oben beschrieben, so wird das Lithium bevorzugt als Lithiumverbindung auf den Träger aufgebracht. Hier ist im Prinzip jegliche geeignete Lithiumverbindung verwendbar. Bevorzugt wird Lithium in Form von Lithiumnitrat aufgebracht.

Enthält der Katalysator Cäsium als Promotor, wie oben beschrieben, so wird das Cäsium bevorzugt als Cäsiumverbindung auf den Träger aufgebracht. Hier ist im Prinzip jegliche geeignete Cäsiumverbindung verwendbar. Bevorzugt wird Cäsium in Form von Cäsiumhydroxid aufgebracht.

Enthält der Katalysator Schwefel als Promotor, wie oben beschrieben, so wird der Schwefel bevorzugt als Schwefelverbindung auf den Träger aufgebracht. Hier ist im Prinzip jegliche geeignete Schwefelverbindung verwendbar. Bevorzugt wird Schwefel in Form von Ammoniumsulfat aufgebracht.

Bevorzugt wird der mindestens eine weitere Promotor, weiter bevorzugt die mindestens eine weitere Promotorverbindung, vor dem Aufbringen in einer geeigneten Lösung, bevorzugt in Wasser, gelöst. Der Träger wird dann bevorzugt mit der erhaltenen Lösung, umfassend einen oder mehrere der weiteren Promotoren, imprägniert. Sollten mehrere weitere Promotoren zugegeben werden, können diese in einem einzigen Imprägnierungsschritt oder in mehreren Imprägnierungsschritten entweder zusammen oder separat auf den Träger aufgebracht werden. Was die Lösung, umfassend einen oder mehrere der weiteren Promotoren, betrifft, so kann diese auf jede geeignete Weise hergestellt werden. Beispielsweise können die Promotoren jeweils separat in jeweils einer Lösung gelöst werden und die erhaltenen Lösungen, enthaltend jeweils einen Promotor, anschließend zur Imprägnierung eingesetzt werden. Ebenfalls ist es möglich, dass zwei oder mehrere der weiteren Promotoren zusammen in einer Lösung gelöst werden, und die erhaltende Lösung anschließend zur Imprägnierung eingesetzt wird. Zudem ist es möglich, dass die erhaltenen Lösungen, enthaltend mindestens einen Promotor, vor dem Imprägnieren vereinigt werden und die erhaltene Lösung, enthaltend alle Promotoren, auf dem Träger aufgebracht wird.

Werden beispielsweise mindestens Rhenium als Promotor und Cäsium, Wolfram, Lithium, Schwefel als weitere Promotoren eingesetzt, so wird gemäß einer besonders bevorzugten Ausführungsform, mindestens eine Lösung enthaltend Cäsium und Wolfram, eine weitere Lösung enthaltend Lithium und Schwefel, und eine weitere Lösung enthaltend Rhenium, hergestellt. Die Lösungen werden entweder in separaten Imprägnierungsschritten auf dem Träger aufgebracht, oder vor dem Aufbringen zu einer Lösung vereinigt und erst danach zur Imprägnierung verwendet. Bevorzugt werden die Lösungen bzw. die vereinigte Lösung zusammen mit G2 aufgebracht.

Besonders bevorzugt werden die Lösungen mit einer mindestens eine Silberverbindung enthaltenden Lösung unter Erhalt des Gemisches G2 vereinigt. Somit umfasst G2 besonders bevorzugt neben der mindestens einen Silberverbindung, mindestens eine Rheniumverbindung, mindestens eine Cäsiumverbindung, mindestens eine Lithiumverbindung, mindestens eine Wolframverbindung und ggf. weitere Promotoren, jeweils in Form einer Verbindung.

Demnach betrifft die vorliegende Erfindung auch ein Verfahren, wie oben beschrieben, und einen Katalysator, herstellbar oder hergestellt nach diesem Verfahren, wobei der Katalysator zusätzlich mindestens einen weiteren Promotor ausgewählt aus Elementen der Gruppe IA, VIB, VIIB und VIA des Periodensystems der Elemente, bevorzugt ausgewählt aus der Gruppe bestehend aus Wolfram, Lithium, Schwefel, Cäsium, Chrom, Mangan, Molybdän und Kalium enthält, wobei der mindestens eine weitere Promotor bevorzugt in Schritt (iv) auf dem getrockneten und/oder calcinierten Träger durch Imprägnieren mit dem Gemisch G2, welches zusätzlich den mindestens einen Promotor umfasst, aufgebracht wird.

Gemäß einer besonders bevorzugten Ausführungsform enthält der Katalysator Rhenium in einer Menge von 150 bis 450 ppm, Wolfram in einer Menge von 10 bis 300 ppm, Cäsium in einer Menge 50 bis 700 ppm, Lithium in einer Menge von 50 bis 300 ppm und Schwefel in einer Menge von 5 bis 150 ppm.

Demnach betrifft die vorliegende Erfindung auch ein Verfahren, wie oben beschrieben, und einen Katalysator, herstellbar oder hergestellt nach diesem Verfahren, wobei der Katalysator Rhenium als Promotor in einer Menge von 150 bis 450 ppm, und als weitere Promotoren mindestens Wolfram in einer Menge von 10 bis 300 ppm, Cäsium in einer Menge von 50 bis 700 ppm, Lithium in einer Menge von 50 bis 300 ppm und Schwefel in einer Menge von 5 bis 150 ppm enthält.

### Verfahren zur Herstellung von Ethylenoxid

Die erfindungsgemäßen Katalysatoren bzw. die nach einem erfindungsgemäßen Verfahren erhältlichen Katalysatoren eignen sich insbesondere als Katalysatoren für die Herstellung von Ethylenoxid aus Ethylen, umfassend eine Oxidation von Ethylen. Es werden hohe Selektivitäten, insbesondere vorteilhafte Startselektivitäten, und gute Aktivitäten erreicht.

Die vorliegende Erfindung betrifft daher gemäß einem weiteren Aspekt auch ein Verfahren zur Herstellung von Ethylenoxid aus Ethylen, umfassend eine Oxidation von Ethylen in Gegenwart eines Katalysators für die Herstellung von Ethylenoxid, wie oben beschrieben.

Darüber hinaus betrifft die vorliegende Erfindung auch die Verwendung eines Katalysators, wie oben beschrieben, für die Herstellung von Ethylenoxid.

Erfindungsgemäß kann die Epoxidation nach allen dem Fachmann bekannten Verfahren erfolgen. Dabei können alle in den Ethylenoxid-Herstellungsverfahren des Standes der Technik verwendbaren Reaktoren verwendet werden, beispielsweise außen gekühlte Rohrbündelreaktoren (vgl. Ullmann's Encyclopedia of Industrial Chemistry, 5. Ausgabe, Bd. A-10, S. 117-135, 123-125, VCH-Verlagsgesellschaft, Weinheim 1987) oder auch Reaktoren mit loser Katalysatorschüttung und Kühlrohren, beispielsweise die in DE-A 3414717, EP-A 0 082 609 und EP-A 0 339 748 beschriebenen Reaktoren. Bevorzugt erfolgt die Epoxidation in mindestens einem Rohrreaktor, bevorzugt in einem Rohrbündelreaktor.

Zur Herstellung von Ethylenoxid aus Ethylen und Sauerstoff kann erfindungsgemäß unter herkömmlichen Reaktionsbedingungen, wie sie beispielsweise in DE 25 21 906 A1, EP 0 014 457 A2, DE 2 300 512 A1, EP 0 172 565 A2, DE 24 54 972 A1, EP 0 357 293 A1, EP 0 266 015 A1, EP 0 085 237 A1, EP 0 082 609 A1 und EP 0 339 748 A2 beschrieben sind, gearbeitet werden. Dem Ethylen und molekularen Sauerstoff enthaltenden Reaktionsgas können dabei zusätzlich noch Inertgase wie Stickstoff oder sich unter den Reaktionsbedingungen inert verhaltende Gase wie Wasserdampf, Methan sowie gegebenenfalls Reaktionsmoderatoren, beispielsweise Halogenide, Kohlenwasserstoffe wie Ethylchlorid, Vinylchlorid oder 1,2-Dichlorethan zugemischt werden. Zweckmäßigerweise liegt der Sauerstoffgehalt des Reaktionsgases in einem Bereich, in dem keine explosionsfähigen Gasgemische vorliegen. Eine geeignete Zusammensetzung des Reaktionsgases zur Herstellung von Ethylenoxid kann z. B. eine Menge an Ethylen im Bereich von 10 bis 80 Vol.-%, bevorzugt von 20 bis 60 Vol.-%, weiter bevorzugt von 25 bis 50 Vol.-%, und besonders bevorzugt im Bereich von 30 bis 40 Vol.-%, bezogen auf das Gesamtvolumen des Reaktionsgases, umfassen. Der Sauerstoffgehalt des Reaktionsgases liegt dabei zweckmäßigerweise in dem Bereich von höchstens 10 Vol.-%, bevorzugt von höchstens 9 Vol.-%, weiter bevorzugt von höchstens 8 Vol.-%, und ganz besonders bevorzugt von höchstens 7 Vol.-%, bezogen auf das Gesamtvolumen des Reaktionsgases.

Bevorzugt enthält das Reaktionsgas einen chlorhaltigen Reaktionsmoderator wie Ethylchlorid, Vinylchlorid oder Dichlorethan in einer Menge von 0 bis 15 ppm, bevorzugt in einer Menge von 0,1 bis 8 ppm. Der Rest des Reaktionsgases besteht in der Regel aus Kohlenwasserstoffen, wie beispielsweise Methan, oder aber aus Inertgasen wie Stickstoff. Zusätzlich können auch noch andere Stoffe wie Wasserdampf, Kohlendioxid oder Edelgase im Reaktionsgas enthalten sein.

Die oben beschriebenen Bestandteile des Reaktionsgemisches können gegebenenfalls jeweils geringe Mengen an Verunreinigungen aufweisen. Ethylen kann beispielsweise in jeglicher Reinheitsstufe, die für die erfindungsgemäße Gasphasenoxidation geeignet ist, verwendet werden. Geeignete Reinheitsstufen schließen ein, sind aber nicht limitiert auf, Polymer-Grade-Ethylen, welches typischerweise eine Reinheit von mindestens 99 % hat und Chemical-Grade-Ethylen, welches eine geringe Reinheit von typischerweise weniger als 95 % aufweist. Die Verunreinigungen bestehen typischerweise vor allem aus Ethan, Propan und/oder Propen.

Die Umsetzung bzw. Oxidation von Ethylen zu Ethylenoxid wird üblicherweise bei erhöhter Temperatur durchgeführt. Bevorzugt sind Temperaturen im Bereich von 150 bis 350 °C, weiter bevorzugt im Bereich von 180 bis 300 °C, weiter bevorzugt im Bereich von 190 °C bis 280 °C, und besonders bevorzugt im Bereich von 200 °C bis 280 °C. Demnach betrifft die vorliegende Erfindung auch ein Verfahren, wie oben beschrieben, wobei die Oxidation bei einer Temperatur im Bereich von 180 bis 300 °C, bevorzugt im Bereich von 200 bis 280 °C, erfolgt.

Bevorzugt wird bei der erfindungsgemäßen Umsetzung (Oxidation) bei Drücken im Bereich von 5 bar bis 30 bar gearbeitet. Weiter bevorzugt erfolgt die Oxidation bei einem Druck im Bereich von 5 bar bis 25 bar, bevorzugt bei einem Druck im Bereich von 10 bar bis 20 bar und insbesondere im Bereich von 14 bar bis 20 bar. Demnach betrifft die vorliegende Erfindung auch ein Verfahren, wie oben beschrieben, wobei die Oxidation bei einem Druck im Bereich von 14 bar bis 20 bar erfolgt.

Bevorzugt wird die Oxidation in einem kontinuierlichen Verfahren durchgeführt. Wird die Reaktion kontinuierlich durchgeführt, so wird eine GHSV (gas hourly space velocity) in Abhängigkeit von der Art des gewählten Reaktors, beispielsweise von der Größe/Durchschnittsfläche des Reaktors, der Form und der Größe des Katalysators verwendet, die bevorzugt im Bereich von 800 bis 10000/h, bevorzugt im Bereich von 2000 bis 6000/h, weiter bevorzugt im Bereich von 2500 bis 5000/h, liegt, wobei sich die Angaben auf das Volumen des Katalysators beziehen.

Vorteilhafterweise kann die Herstellung von Ethylenoxid aus Ethylen und Sauerstoff in einem Kreisprozess durchgeführt werden. Dabei wird das Reaktionsgemisch im Kreislauf durch den Reaktor geleitet, wo nach jedem Durchgang das neu gebildete Ethylenoxid sowie die bei der Umsetzung gebildeten Nebenprodukte aus dem Produktgasstrom entfernt werden, der nach Ergänzung mit den erforderlichen Mengen an Ethylen, Sauerstoff und Reaktionsmoderatoren wieder in den Reaktor zugeführt wird. Die Abtrennung des Ethylenoxids aus dem Produktgasstrom und seiner Aufarbeitung können nach den üblichen Verfahren des Standes der Technik (vgl. Ullmann's Encyclopedia of Industrial Chemistry, 5. Ausgabe, Bd. A-10, S. 117-135, 123-125, VCH-Verlagsgesellschaft, Weinheim 1987) erfolgen.

Im Folgenden werden besonders bevorzugte Ausführungsformen der Erfindung genannt, wobei die sich durch die angegebenen Rückbezüge ergebenden Kombinationen explizit mit umfasst sind.
1. Verfahren zur Herstellung eines Katalysators für die Herstellung von Ethylenoxid, umfassend Silber und Zink aufgebracht auf einem Träger, wobei das Verfahren mindestens umfasst
   (i) Bereitstellen des Trägers,
   (ii) Aufbringen von Zink auf dem Träger gemäß (i) in einer Menge von 10 ppm bis 1500 ppm, jeweils berechnet als Element und bezogen auf das Gesamtgewicht des Trägers gemäß (i), durch Inkontaktbringen des Trägers mit mindestens einem Gemisch G1 umfassend mindestens eine Zinkverbindung,
   (iii) Trocknen und/oder Calcinieren des gemäß (ii) erhaltenen Trägers,
   (iv) Aufbringen von Silber auf dem getrockneten und/oder calcinierten Träger durch Inkontaktbringen des Trägers mit mindestens einem Gemisch G2 umfassend mindestens eine Silberverbindung.
2. Verfahren nach Ausführungsform 1, wobei der Katalysator mindestens Rhenium als Promotor umfasst, bevorzugt in einer Menge von 50 ppm bis 1000 ppm, bezogen auf das Gesamtgewicht des Katalysators und berechnet als Element.
3. Verfahren nach Ausführungsform 2, wobei das Rhenium in Schritt (iv) auf dem getrockneten und/oder calcinierten Träger gemäß (iii) durch Imprägnieren mit dem Gemisch G2, welches zusätzlich mindestens eine Rheniumverbindung umfasst, aufgebracht wird.
4. Verfahren nach einer der Ausführungsformen 1 bis 3, wobei der Träger ein Aluminiumoxidträger, bevorzugt ein alpha-Aluminiumoxidträger ist.
5. Verfahren nach Ausführungsform 4, wobei der Aluminiumoxidträger ein alpha-Aluminumoxidträger ist mit einer Reinheit, bezogen alpha-Aluminiumoxid, von mindestens 85 %.
6. Verfahren nach Ausführungsform 5, wobei der Aluminiumoxidträger Calcium in einer Menge im Bereich von 10 bis 1500 ppm, bezogen auf das Gesamtgewicht des Trägers und berechnet als Element, enthält.
7. Verfahren nach Ausführungsform 5 oder 6, wobei der Aluminiumoxidträger Magnesium in einer Menge im Bereich von höchstens 800 ppm, bevorzugt in einer Menge von 1 bis 500 ppm, bezogen auf das Gesamtgewicht des Trägers und berechnet als Element, enthält.
8. Verfahren nach einer der Ausführungsformen 5 bis 7, wobei der Aluminiumoxidträger Kalium in einer Menge im Bereich von höchstens 1000 ppm, bezogen auf das Gesamtgewicht des Trägers und berechnet als Element, enthält.
9. Verfahren nach einer der Ausführungsformen 5 bis 8, wobei der Aluminiumoxidträger Natrium in einer Menge im Bereich von 10 bis 1500 ppm, bezogen auf das Gesamtgewicht des Trägers und berechnet als Element, enthält.
10. Verfahren nach einer der Ausführungsformen 5 bis 9, wobei der Aluminiumoxidträger Silicium in einer Menge im Bereich von 50 bis 10000 ppm, weiter bevorzugt in einer Menge von 100 bis 5000 ppm, weiter bevorzugt in einer Menge von 1000 bis 2500 ppm, bezogen auf das Gesamtgewicht des Trägers und berechnet als Element, enthält.
11. Verfahren nach einer der Ausführungsformen 5 bis 10, wobei der Aluminiumoxidträger Zirkon in einer Menge im Bereich von 1 bis 10000 ppm, bezogen auf das Gesamtgewicht des Trägers und berechnet als Element, enthält.
12. Verfahren nach einer der Ausführungsformen 1 bis 11, wobei der Träger eine BET-Oberfläche, bestimmt gemäß DIN ISO 9277, im Bereich von 0,5 bis 1,5 m²/g aufweist.
13. Verfahren nach einer der Ausführungsformen 1 bis 12, wobei der Träger eine bimodale Porenverteilung aufweist, bevorzugt eine bimodale Porenverteilung mindestens enthaltend Poren mit Porendurchmessern im Bereich von 0,1 bis 10 µm und Poren mit Porendurchmessern im Bereich von 15 bis 100 µm, bestimmt durch Hg-Porosimetrie gemäß DIN 66133.
14. Verfahren nach einer der Ausführungsformen 1 bis 13, wobei Gemisch G1 Zinkacetat und Ethylendiamin umfasst.
15. Verfahren nach einer der Ausführungsformen 1 bis 14, wobei in (ii) Zink in einer Menge von 50 bis 1250 ppm, berechnet als Element und bezogen auf das Gesamtgewicht des Trägers, gemäß (i) auf den Träger aufgebracht wird.
16. Verfahren nach einer der Ausführungsformen 1 bis 15, wobei der gemäß (i) bereitgestellte Träger zusätzlich 1 bis 1000 ppm Zink und 1 bis 10000 ppm Zirkon, jeweils berechnet als Element und bezogen auf das Gesamtgewicht des Trägers gemäß (i), enthält.
17. Verfahren nach einer der Ausführungsformen 1 bis 16, wobei der Katalysator zusätzlich mindestens einen weiteren Promotor ausgewählt aus Elementen der Gruppe IA, VIB, VIIB und VIA des Periodensystems der Elemente, bevorzugt ausgewählt aus der Gruppe bestehend aus Wolfram, Lithium, Schwefel, Cäsium, Chrom, Mangan, Molybdän und Kalium enthält, wobei der mindestens eine Promotor bevorzugt in Schritt (iv) auf dem getrockneten und/oder calcinierten Träger gemäß (iii) durch Imprägnieren mit dem Gemisch G2, welches zusätzlich den mindestens einen Promotor umfasst, aufgebracht wird.
18. Verfahren nach einer der Ausführungsformen 1 bis 17, wobei der Katalysator Rhenium in einer Menge von 150 bis 450 ppm, Wolfram in einer Menge von 10 bis 300 ppm, Cäsium in einer Menge 50 bis 700 ppm, Lithium in einer Menge von 50 bis 300 ppm und Schwefel in einer Menge von 5 bis 150 ppm, enthält.
19. Verfahren nach einer der Ausführungsformen 1 bis 18, wobei der Katalysator Silber in einer Menge von 5 bis 35 Gew.-%, berechnet als Element und bezogen auf das Gesamtgewicht des Katalysators, enthält.
20. Verfahren nach einer der Ausführungsformen 1 bis 19, weiterhin umfassend (v) Trocknen und/oder Calcinieren des Trägers gemäß (iv).
21. Katalysator für die Herstellung von Ethylenoxid, mindestens umfassend Silber und Zink aufgebracht auf einem Träger, herstellbar oder hergestellt durch ein Verfahren nach einer der Ausführungsformen 1 bis 20.
22. Verfahren zur Herstellung von Ethylenoxid aus Ethylen umfassend eine Oxidation von Ethylen in Gegenwart eines Katalysators nach Ausführungsform 21.
23. Verwendung eines Katalysators nach Ausführungsform 21, als Katalysator für die Herstellung von Ethylenoxid aus Ethylen durch Oxidation von Ethylen.

Im Folgenden wird die vorliegende Erfindung anhand von Beispielen näher erläutert.

### Beispiele:

### 1. Allgemeine Vorschrift zur erfindungsgemäßen Katalysatorherstellung

### 1.1 Verwendeter Träger

Es wurde ein bimodaler alpha-Aluminiumoxidträger mit Hohlring-Geometrie (6,10 x 6,31 x 2,87) mit 400 ppm Ca, 60 ppm Na, 2200 ppm Si, 5000 ppm Zirkon, 200 ppm Zink und weniger als 50 ppm an Eisen, Magnesium und Kalium mit einer BET-Oberfläche von 0,89 m²/g und einer Vakuumkaltwasseraufnahme von 0,47 ml/g verwendet.

### 1.2 Herstellen und Aufbringen der Zinkkomplexlösung auf dem Träger

Der Träger wurde mit einer Zinkkomplexlösung enthaltend Zinkacetat und Ethylendiamin, gelöst in Wasser, imprägniert. Hierzu wurden zunächst 180,2 g Ethylendiamin in einer 500 ml Rührapparatur ohne Inertgasspülung zusammen mit 180,2 g Wasser vorgelegt und mit Eiswasserkühlung auf 10°C temperiert. Anschließend wurden langsam 250,1 g Zinkacetat-dihydrat zugegeben und unter Kühlung für 2 h nachgerührt. Es wurden 611,78 g Zinkkomplexlösung mit 12,2 Gew.-% Zink Massenanteil erhalten.

Die Imprägnierung erfolgte mittels Vakuumimprägnierung. Hierzu wurde zunächst der alpha-Aluminiumoxidträger bei einem Druck von 6 mbar und einer Temperatur von 22°C in einem Rotationsverdampfer evakuiert und anschließend die Zinkkomplexlösung aufgetropft. Anschließend wurde der imprägnierte Träger weitere 15 min im Vakuum rotieren gelassen. Danach wurde der Träger 1 h bei Raumtemperatur und Normaldruck in der Apparatur belassen und alle 15 min leicht durchmischt.

Nach der Imprägnation mit Zink erfolgte eine Kalzination bei 280°C für etwa 12 min unter Luft, um die organischen Anteile der Komplexlösungen zu zersetzen und das Metall auf dem Träger abzuscheiden. Nach Abkühlung auf Raumtemperatur wurde der so mit Zink vorbehandelte Träger in einen Muffelofen überführt und innerhalb von 8 h auf 1000°C erhitzt, um dann für die Dauer von 2 h bei 1000°C kalziniert zu werden. Nach Ablauf der 2 h wurde der Träger im Ofen auf Raumtemperatur abgekühlt.

### 1.3 Herstellung der Silberkomplexlösung

Es wurden 1,5 L Wasser vorgelegt und unter Rühren 550 g Silbernitrat zugeben und darin vollständig aufgelöst. Dabei wurde die Lösung auf 40°C erwärmt. 402,62 g Kalilauge (47.8%) wurden mit 1,29 L Wasser gemischt. Anschließend wurden 216,31 g Oxalsäure zugegeben und vollständig gelöst und die Lösung auf 40°C erwärmt. Die Kaliumoxalat-Lösung wurde anschließend innerhalb von ca. 45 min (Volumenstrom = ca. 33 ml/min) zur Silbernitrat-Lösung (40°C) mit Hilfe einer Dosierpumpe zugeben. Nach kompletter Zugabe wurde die erhaltene Lösung noch 1 h bei 40°C nachrühren gelassen. Das gefällte Silberoxalat wurde abfiltriert und der erhaltene Filterkuchen solange mit 1 L-Wasserportionen gewaschen (ca 10 L), bis er Kalium- bzw. Nitrat-frei war (bestimmt mittels Leitfähigkeitsmessung der Waschlösung, Kalium- bzw. Nitrat-frei bedeutet vorliegend eine Leitfähigkeit < 40 µS/cm). Das Wasser wurde möglichst vollständig aus dem Filterkuchen entfernt und die Restfeuchte des Filterkuchens bestimmt. Es wurden 620 g Silberoxalat mit einem Wassergehalt von 20,80% erhalten.

309,7 g Ethylendiamin wurden mit einem Eisbad auf ca. 10°C abgekühlt und 247,9 g Wasser in kleinen Portionen zugeben. Nach beendeter Wasserzugabe wurden die 620 g des erhaltenen feuchten Silberoxalates (das entspricht 491,0 g trockenem Silberoxalat) innerhalb von ca. 30 min in kleinen Portionen zugeben. Das Gemisch wurde über Nacht bei RT gerührt und der Rückstand anschließend abzentrifugiert. Von der verbleibenden klaren Lösung wurde der Ag-Gehalt refraktometrisch und die Dichte mit Hilfe eines 10 mL Messzylinders bestimmt.

Die erhaltene Lösung enthielt 29.35 Gew.-% Silber, berechnet als Element, und hatte eine Dichte von 1,536 g/mL.

### 1.4 Allgemeine Vorschrift zur Herstellung der Lösung enthaltend Silber und Promotoren

Die gemäß Vorschrift 1.3 hergestellte Silberoxalatlösung wurde vorgelegt. Dazu wurden eine wässrige Lösung aus Lithium und Schwefel (Lithiumnitrat und (NH₄)₂SO₄), eine wässrige Lösung aus Wolfram und Cäsium (H₂WO₄ und CsOH) und eine wässrige Lösung aus Rhenium (Ammoniumperrhenat) zugegeben und die Lösung 5 min gerührt.

### 1.5 Aufbringen der Lösung auf dem Träger

120 g des mit Zink imprägnierten Trägers gemäß allgemeiner Vorschrift 1.2 (siehe Tabelle 1) wurden in einem Rotationsverdampfer vorgelegt und bei 6 mbar, evakuiert. Der Träger wurde für ca. 10 min vorevakuiert.

Die gemäß allgemeiner Vorschrift 1.4 erhaltene Lösung wurde innerhalb von 15 min auf den Träger aufgetropft und anschließend wurde der imprägnierte Träger weitere 15 min im Vakuum rotieren gelassen. Danach wurde der Träger 1 h bei Raumtemperatur und Normaldruck in der Apparatur belassen und alle 15 min leicht durchmischt.

### 1.6 Kalzination des imprägnierten Trägers

Der imprägnierte Träger wurde für 12 min bei 283°C unter 8,3 m³ Luft pro Stunde in einem Umluftofen (Firma HORO, Typ 129 ALV-SP, Fabr.Nr.:53270) behandelt.

Für alle hergestellten Katalysatoren ist die Menge an Silber und der Promotoren identisch.

### 1.7 Epoxidation

Die Epoxidation wurde in einem Versuchsreaktor bestehend aus einem senkrecht stehenden Reaktionsrohr aus Edelstahl mit einem Innendurchmesser von 6 mm und einer Länge von 2200 mm durchgeführt. Das mit einem Mantel versehene Reaktionsrohr wurde mit heißem Öl der Temperatur T, das durch den Mantel strömte, beheizt. Mit sehr guter Näherung entspricht die Temperatur des Öls der Temperatur im Reaktionsrohr und somit der Reaktionstemperatur. Das Reaktionsrohr wurde von unten nach oben auf einer Höhe von 212 mm mit inerten Steatitkugeln (1,0 - 1,6 mm), darüber auf einer Höhe von 1100 mm mit 38,2 g Katalysatorsplitt, Partikelgröße 0,5-0,9 mm, und darüber auf einer Höhe von 707 mm mit inerten Steatitkugeln (1,0 - 1,6 mm) gefüllt. Das Eingangsgas trat von oben in den Reaktor ein und am unteren Ende nach Passieren der Katalysatorschüttung wieder aus.

Das Eingangsgas bestand aus 35 Vol.-% Ethylen, 7 Vol.-% Sauerstoff, 1 Vol.-% CO₂ (EC (Ethylenchlorid)-Moderation). Zu Beginn wurden 2,5 ppm EC zum Anfahren verwendet. Je nach Katalysator und Performance wurde die EC-Konzentration alle 24 h bis maximal 8 ppm erhöht. Der Rest des Eingangsgases bestand aus Methan. Die Versuche wurden bei einem Druck von 15 bar und einer Gasbelastung (GHSV) von 4750/h sowie einer Raum-Zeit-Ausbeute von 250 kg EO/(m³(Kat) x h) durchgeführt.

Die Reaktionstemperatur wurde gemäß der vorgegebenen Ethylenoxid-Abgaskonzentration von 2,7 % geregelt. Zur Optimierung des Katalysators im Hinblick auf Selektivität und Umsatz wurden zwischen 2,2 und 8,0 ppm Ethylenchlorid als Moderator zum Eingangsgas zudosiert.

Das am Reaktor austretende Gas wurde mittels Online-MS analysiert. Aus den Analyseergebnissen wurde die Selektivität ermittelt.

### Beispiel 1

170 g des Trägers gemäß 1.1 wurden gemäß Vorschrift 1.2 mit 1,6560 g der Zinkkomplexlösung (12,2 Gew.-% Zn) und 78,26 g Wasser imprägniert. Vor der Herstellung und Zugabe der Lösung gemäß 1.4 wurde die Wasseraufnahme des Trägers erneut bestimmt. Diese neue Wasseraufnahme betrug 0,471 ml/g und diente zur Berechnung der zur Herstellung gemäß 1.4 benötigten Mengen.

Folgende Mengen wurden bei der Herstellung der Lösung gemäß allgemeiner Vorschrift 1.4 eingesetzt:
89,9527 g Silberoxalatlösung (28,62% Ag)
1,1127 g einer Lösung mit 2,85% Lithium und 0,21% Schwefel,
3,3228 g einer Lösung mit 1,00% Wolfram und 1,75% Cäsium,
1,6630 g einer Lösung mit 3,1% Rhenium,
0,7699 g Wasser.

Der gemäß allgemeiner Vorschrift 1.2 erhaltene Träger wurde anschließend gemäß Vorschriften 1.5 und 1.6 zum Katalysator umgesetzt. Der erhaltene Katalysator, der 1000 ppm auf den Träger aufgebrachtes Zink, berechnet als Element und bezogen auf das Gesamtgewicht des Trägers, enthielt, wurde anschließend gemäß allgemeiner Vorschrift 1.7 getestet. Das Ergebnis ist Tabelle 1 zu entnehmen.

### Beispiel 2

170 g des Trägers gemäß 1.1 wurden gemäß Vorschrift 1.2 mit 0,7019 g der Zinkkomplexlösung (12,2 Gew.-% Zn) und 79,29 g Wasser imprägniert. Vor der Herstellung und Zugabe der Lösung gemäß 1.4 wurde die Wasseraufnahme des Trägers erneut bestimmt. Diese neue Wasseraufnahme betrug 0,458 ml/g und diente zur Berechnung der Mengen.

Folgende Mengen wurden bei der Herstellung der Lösung gemäß allgemeiner Vorschrift 1.4 eingesetzt:
88,8843 g Silberoxalatlösung (28,93% Ag)
1,1062 g einer Lösung mit 2,85% Lithium und 0,21% Schwefel,
3,3236 g einer Lösung mit 1,00% Wolfram und 1,75 % Cäsium,
1,2561 g einer Lösung mit 4,1% Rhenium,
0,2401 g Wasser.

Der gemäß allgemeiner Vorschrift 1.2 erhaltene Träger wurde anschließend gemäß Vorschriften 1.5 und 1.6 zum Katalysator umgesetzt. Der erhaltene Katalysator, der 500 ppm vor der Imprägnation mit Silber auf den Träger aufgebrachtes Zink, berechnet als Element und bezogen auf das Gesamtgewicht des Trägers, enthielt, wurde anschließend gemäß allgemeiner Vorschrift 1.7 getestet. Das Ergebnis ist Tabelle 1 zu entnehmen.

### Beispiel 3

170 g des Trägers gemäß 1.1 wurden gemäß Vorschrift 1.2 mit 0,4156 g der Zinkkomplexlösung (12,2 Gew.-% Zn) und 79,50 g Wasser imprägniert. Vor der Herstellung und Zugabe der Lösung gemäß 1.4 wurde die Wasseraufnahme des Trägers erneut bestimmt. Diese neue Wasseraufnahme betrug 0,471 ml/g und diente zur Berechnung der Mengen.

Folgende Mengen wurden bei der Herstellung der Lösung gemäß allgemeiner Vorschrift 1.4 eingesetzt:
89,9042 g Silberoxalatlösung (28,62% Ag)
1,1233 g einer Lösung mit 2,85% Lithium und 0,21% Schwefel,
3,3230 g einer Lösung mit 1,00% Wolfram und 1,75 % Cäsium,
1,6600 g einer Lösung mit 4,1 % Rhenium,
0,8947 g Wasser.

Der gemäß allgemeiner Vorschrift 1.2 erhaltene Träger wurde anschließend gemäß Vorschriften 1.5 und 1.6 zum Katalysator umgesetzt. Der erhaltene Katalysator, der 250 ppm vor der Imprägnation mit Silber auf den Träger aufgebrachtes Zink, berechnet als Element und bezogen auf das Gesamtgewicht des Trägers, enthielt, wurde anschließend gemäß allgemeiner Vorschrift 1.7 getestet. Das Ergebnis ist Tabelle 1 zu entnehmen.

### Beispiel 4 (nicht erfindungsgemäß)

140 g des Trägers gemäß 1.1 wurden gemäß Vorschrift 1.2 mit 72,2723 g der Zinkkomplexlösung (12,2 Gew.-% Zn) und 6,4738 g Wasser imprägniert. Vor der Herstellung und Zugabe der Lösung gemäß 1.4 wurde die Wasseraufnahme des Trägers erneut bestimmt. Diese neue Wasseraufnahme betrug 0,435 ml/g und dient zur Berechnung der Mengen.

Folgende Mengen wurden bei der Herstellung der Lösung gemäß allgemeiner Vorschrift 1.4 eingesetzt:
74,6531 g der Silberoxalatlösung (29,35% Ag),
1,0018 g einer wässrigen Lösung aus Lithium und Schwefel (2,85% Lithium aus Lithiumnitrat und 0,21% Schwefel aus (NH₄)₂SO₄),
1,4966 g einer wäßrigen Lösung aus Wolfram und Cäsium (2,00 % Wolfram aus H₂WO₄ und 3,50 % Cäsium aus CsOH),
1,1365 g einer wäßrigen Lösung aus Rhenium (4,1 % Rhenium aus Ammoniumperrhenat).

Der gemäß allgemeiner Vorschrift 1.2 erhaltene Träger wurde anschließend gemäß Vorschriften 1.5 und 1.6 zum Katalysator umgesetzt. Der erhaltene Katalysator, der 5 Gew.-% vor der Imprägnation mit Silber auf den Träger aufgebrachtes Zink, berechnet als Element und bezogen auf das Gesamtgewicht des Trägers, enthielt, wurde anschließend gemäß allgemeiner Vorschrift 1.7 getestet. Das Ergebnis ist Tabelle 1 zu entnehmen.

### Beispiel 5 (nicht erfindungsgemäß)

Der Katalysator wurde gemäß allgemeiner Vorschrift 1.5 bis 1.6 hergestellt, wobei der Imprägnationslösung, wie in 1.4 beschrieben, zusätzlich 0,6835 g der Zinkkomplexlösung (12,2 Gew.-% Zn) und 0,6408 g Wasser zugesetzt wurden. Es wurden 140 g Träger mit einer Wasseraufnahme von 0,47 ml/g eingesetzt.

Eine vorgelagerte Imprägnierung und Kalzination mit Zink gemäß allgemeiner Vorschrift 1.2 bis 1.3 erfolgte nicht.

Neben 0,6835 g der Zinkkomplexlösung (12,2 Gew.-% Zn) und 0,6408 g Wasser wurden folgende Mengen bei der Herstellung der Lösung gemäß allgemeiner Vorschrift 1.4 eingesetzt:
88,9344 g Silberoxalatlösung (28,93% Ag)
1,1113 g einer Lösung mit 2,85% Lithium und 0,21% Schwefel,
3,3216 g einer Lösung mit 1,00% Wolfram und 1,75 % Cäsium,
1,6631 g einer Lösung mit 3,1% Rhenium.

Der erhaltene Katalysator, der 500 ppm Zink zusammen mit Silber und den anderen Promotoren auf den Träger aufgebracht, berechnet als Element und bezogen auf das Gesamtgewicht des Trägers, enthielt wurde anschließend gemäß allgemeiner Vorschrift 1.7 getestet. Das Ergebnis ist Tabelle 1 zu entnehmen.

### Beispiel 6 (nicht erfindungsgemäß)

Der Katalysator wurde gemäß allgemeiner Vorschrift 1.5 bis 1.6 hergestellt. Eine Imprägnierung mit Zink gemäß allgemeiner Vorschrift 1.2 bis 1.3 erfolgte nicht.

Es wurden folgende Mengen bei der Herstellung der Lösung gemäß allgemeiner Vorschrift 1.4 eingesetzt:
59,4199 g Silberoxalatlösung (29,04% Ag)
0,7841 g einer Lösung mit 2,85 % Lithium und 0,21% Schwefel
1,1751 g einer Lösung mit 2,00% Wolfram und 3,50 % Cäsium,
1,1749 g einer Lösung mit 3,1% Rhenium, 4,9638 g Wasser.

Mit dieser Lösung wurden 100 g Träger mit einer Wasseraufnahme 0,47 ml/g gemäß Vorschrift 1.5 und 1.6 behandelt. Der erhaltene Katalysator wurde anschließend gemäß allgemeiner Vorschrift 1.7 getestet. Das Ergebnis ist Tabelle 1 und Figur 1 zu entnehmen.

### Ergebnisse

Zusammensetzung der Katalysatoren gemäß Beispiel 1 bis 6: Die Katalysatoren enthalten 14.8 % Ag, 190 ppm Li, 14 ppm S, 200 ppm W, 350 ppm Cs, 310 ppm Re und, wie angegeben, verschiedene Mengen an Zink.

Es konnte gezeigt werden, dass eine Imprägnierung des Trägers mit Zinkkomplexlösung und anschließender Kalzination vor Imprägnierung mit Silber einen positiven Effekt auf die Startselektivität hervorruft. Im Vergleich zum nicht mit Zink vorimprägnierten Katalysator führt der Zusatz von Zink zu einer besseren "Start of Run" Selektivität von bis zu 3,3%, bei sonst nahezu identischem katalytischen Verhalten. Bei zu hoher Zinkkonzentration oder gleichzeitigem Aufbringen von Zink mit den anderen Bestandteilen des Katalysators nimmt die Selektivität des Katalysators deutlich ab (Beispiele 4 und 5).

**Tabelle 1: Katalyseergebnisse Beispiele 1 bis 6**

| | **Zinkquelle** | **Start of Run Selektivität / %** | **Temperatur / °C** |
|---|---|---|---|
| Beispiel 1 | Komplexlösung 1000 ppm | 88.4 % | 237 |
| Beispiel 2 | Komplexlösung 500 ppm | 88.1 % | 239 |
| Beispiel 3 | Komplexlösung 250 ppm | 87.5 % | 237 |
| Beispiel 4 | Komplexlösung 5% | 79.5 % | 229 |
| Beispiel 5 | Komplexlösung 500 ppm | 82.9 % | 236 |
| Beispiel 6 | ----- | 85.1 % | 238 |

## Patentansprüche

1. Verfahren zur Herstellung eines Katalysators für die Herstellung von Ethylenoxid, umfassend Silber und Zink, aufgebracht auf einem Träger, wobei das Verfahren mindestens umfasst
(i) Bereitstellen des Trägers,
(ii) Aufbringen von Zink auf dem Träger gemäß (i) in einer Menge von 10 ppm bis 1500 ppm, jeweils berechnet als Element und bezogen auf das Gesamtgewicht des Trägers gemäß (i), durch Inkontaktbringen des Trägers mit mindestens einem Gemisch G1 umfassend mindestens eine Zinkverbindung,
(iii) Trocknen und/oder Calcinieren des gemäß (ii) erhaltenen Trägers,
(iv) Aufbringen von Silber auf dem getrockneten und/oder calcinierten Träger gemäß (iii) durch Inkontaktbringen des Trägers mit mindestens einem Gemisch G2 umfassend mindestens eine Silberverbindung.

2. Verfahren nach Anspruch 1, wobei der Katalysator mindestens Rhenium als Promotor umfasst, bevorzugt in einer Menge von 50 ppm bis 1000 ppm, bezogen auf das Gesamtgewicht des Katalysators und berechnet als Element.

3. Verfahren nach Anspruch 2, wobei das Rhenium in Schritt (iv) auf dem getrockneten und/oder calcinierten Träger gemäß (iii) durch Imprägnieren mit dem Gemisch G2, welches zusätzlich mindestens eine Rheniumverbindung umfasst, aufgebracht wird.

4. Verfahren nach einem der Ansprüche 1 bis 3, wobei der Träger ein Aluminiumoxidträger, bevorzugt ein alpha-Aluminiumoxidträger ist.

5. Verfahren nach einem der Ansprüche 1 bis 4, wobei der Träger eine BET-Oberfläche, bestimmt gemäß DIN ISO 9277, im Bereich von 0,5 bis 1,5 m²/g aufweist.

6. Verfahren nach einem der Ansprüche 1 bis 5, wobei der Träger eine bimodale Porenverteilung aufweist, bevorzugt eine bimodale Porenverteilung mindestens enthaltend Poren mit Porendurchmessern im Bereich von 0,1 bis 10 µm und Poren mit Porendurchmessern im Bereich von 15 bis 100 µm, bestimmt durch Hg-Porosimetrie gemäß DIN 66133.

7. Verfahren nach einem der Ansprüche 1 bis 6, wobei das Gemisch G1 Zinkacetat und Ethylendiamin umfasst.

8. Verfahren nach einem der Ansprüche 1 bis 7, wobei in (ii) Zink in einer Menge von 50 bis 1250 ppm, berechnet als Element und bezogen auf das Gesamtgewicht des Trägers gemäß (i), auf den Träger aufgebracht wird.

9. Verfahren nach einem der Ansprüche 1 bis 8, wobei der gemäß (i) bereitgestellte Trägers zusätzlich 1 bis 1000 ppm Zink und 1 bis 10000 ppm Zirkon, jeweils berechnet als Element und bezogen auf das Gesamtgewicht des Trägers gemäß (i), umfasst.

10. Verfahren nach einem der Ansprüche 1 bis 9, wobei der Katalysator zusätzlich mindestens einen weiteren Promotor ausgewählt aus Elementen der Gruppe IA, VIB, VIIB und VIA des Periodensystems der Elemente, bevorzugt ausgewählt aus der Gruppe bestehend aus Wolfram, Lithium, Schwefel, Cäsium, Chrom, Mangan, Molybdän und Kalium, enthält, wobei der mindestens eine Promotor bevorzugt in Schritt (iv) auf dem getrockneten und/oder calcinierten Träger gemäß (iii) durch Imprägnieren mit dem Gemisch G2, welches zusätzlich den mindestens einen Promotor umfasst, aufgebracht wird.

11. Verfahren nach einem der Ansprüche 1 bis 10, wobei der Katalysator Silber in einer Menge von 5 bis 35 Gew.-%, berechnet als Element und bezogen auf das Gesamtgewicht des Katalysators, enthält.

12. Verfahren nach einem der Ansprüche 1 bis 11, zusätzlich umfassend (v) Trocknen und/oder Calcinieren des gemäß (iv) erhaltenen Trägers.

13. Katalysator für die Herstellung von Ethylenoxid, mindestens umfassend Silber und Zink, aufgebracht auf einem Träger, herstellbar oder hergestellt durch ein Verfahren gemäß einem der Ansprüche 1 bis 12.

14. Verfahren zur Herstellung von Ethylenoxid aus Ethylen umfassend eine Oxidation von Ethylen in Gegenwart eines Katalysators gemäß Anspruch 13.

15. Verwendung eines Katalysators gemäß Anspruch 13 als Katalysator für die Herstellung von Ethylenoxid aus Ethylen durch Oxidation von Ethylen.

## Claims

1. A process for producing a catalyst for preparing ethylene oxide, which comprises silver and zinc applied to a support, where the process comprises at least
(i) providing the support,
(ii) applying zinc to the support according to (i) in an amount of from 10 ppm to 1500 ppm, in each case calculated as element and based on the total weight of the support according to (i), by bringing the support into contact with at least one mixture G1 comprising at least one zinc compound,
(iii) drying and/or calcining the support obtained according to (ii),
(iv) applying silver to the dried and/or calcined support according to (iii) by bringing the support into contact with at least one mixture G2 comprising at least one silver compound.

2. The process according to claim 1, wherein the catalyst comprises at least rhenium as promoter, preferably in an amount of from 50 ppm to 1000 ppm, based on the total weight of the catalyst and calculated as element.

3. The process according to claim 2, wherein the rhenium is applied to the dried and/or calcined support according to (iii) by impregnation with the mixture G2 which additionally comprises at least one rhenium compound in step (iv).

4. The process according to any of claims 1 to 3, wherein the support is an aluminum oxide support, preferably an alpha-aluminum oxide support.

5. The process according to any of claims 1 to 4, wherein the support has a BET surface area determined in accordance with DIN ISO 9277 in the range from 0.5 to 1.5 m²/g.

6. The process according to any of claims 1 to 5, wherein the support has a bimodal pore distribution, preferably a bimodal pore distribution at least comprising pores having pore diameters in the range from 0.1 to 10 µm and pores having pore diameters in the range from 15 to 100 µm, determined by Hg porosimetry in accordance with DIN 66133.

7. The process according to any of claims 1 to 6, wherein the mixture G1 comprises zinc acetate and ethylenediamine.

8. The process according to any of claims 1 to 7, wherein zinc is applied in an amount of from 50 to 1250 ppm, calculated as element and based on the total weight of the support according to (i), to the support in (ii).

9. The process according to any of claims 1 to 8, wherein the support provided according to (i) additionally comprises from 1 to 1000 ppm of zinc and from 1 to 10 000 ppm of zirconium, in each case calculated as element and based on the total weight of the support according to (i).

10. The process according to any of claims 1 to 9, wherein the catalyst additionally comprises at least one further promoter selected from among elements of groups IA, VIB, VIIB and VIA of the Periodic Table of the Elements, preferably selected from the group consisting of tungsten, lithium, sulfur, cesium, chromium, manganese, molybdenum and potassium, where the at least one promoter is preferably applied to the dried and/or calcined support according to (iii) by impregnation with the mixture G2 which additionally comprises the at least one promoter in step (iv).

11. The process according to any of claims 1 to 10, wherein the catalyst comprises silver in an amount of from 5 to 35% by weight, calculated as element and based on the total weight of the catalyst.

12. The process according to any of claims 1 to 11, which further comprises
(v) drying and/or calcining the support obtained according to (iv).

13. A catalyst for preparing ethylene oxide, which comprises at least silver and zinc applied to a support and can be or has been produced by a process according to any of claims 1 to 12.

14. A process for preparing ethylene oxide from ethylene, which comprises oxidation of ethylene in the presence of a catalyst according to claim 13.

15. The use of a catalyst according to claim 13 as catalyst for the preparation of ethylene oxide from ethylene by oxidation of ethylene.

## Revendications

1. Procédé de fabrication d'un catalyseur pour la fabrication d'oxyde d'éthylène, comprenant de l'argent et du zinc, appliqués sur un support, le procédé comprenant au moins :
(i) la mise à disposition du support,
(ii) l'application de zinc sur le support selon (i) en une quantité de 10 ppm à 1 500 ppm, à chaque fois calculée sous forme élémentaire et par rapport au poids total du support selon (i), par mise en contact du support avec au moins un mélange G1 comprenant au moins un composé de zinc,
(iii) le séchage et/ou la calcination du support obtenu selon (ii),
(iv) l'application d'argent sur le support séché et/ou calciné selon (iii) par mise en contact du support avec au moins un mélange G2 comprenant au moins un composé d'argent.

2. Procédé selon la revendication 1, dans lequel le catalyseur comprend au moins du rhénium en tant que promoteur, de préférence en une quantité de 50 ppm à 1 000 ppm, par rapport au poids total du catalyseur et calculée sous forme élémentaire.

3. Procédé selon la revendication 2, dans lequel le rhénium est appliqué à l'étape (iv) sur le support séché et/ou calciné selon (iii) par imprégnation avec le mélange G2, qui comprend en outre au moins un composé de rhénium.

4. Procédé selon l'une quelconque des revendications 1 à 3, dans lequel le support est un support en oxyde d'aluminium, de préférence un support en oxyde d'aluminium alpha.

5. Procédé selon l'une quelconque des revendications 1 à 4, dans lequel le support présente une surface BET, déterminée selon DIN ISO 9277, dans la plage allant de 0, 5 à 1,5 m²/g.

6. Procédé selon l'une quelconque des revendications 1 à 5, dans lequel le support présente une distribution de pores bimodale, de préférence une distribution de pores contenant au moins des pores ayant des diamètres de pores dans la plage allant de 0,1 à 10 µm et des pores ayant des diamètres de pores dans la plage allant de 15 à 100 µm, déterminée par porosimétrie Hg selon DIN 66133.

7. Procédé selon l'une quelconque des revendications 1 à 6, dans lequel le mélange G1 comprend de l'acétate de zinc et de l'éthylènediamine.

8. Procédé selon l'une quelconque des revendications 1 à 7, dans lequel, en (ii), du zinc est appliqué sur le support en une quantité de 50 à 1 250 ppm, calculée sous forme élémentaire et par rapport au poids total du support selon (i).

9. Procédé selon l'une quelconque des revendications 1 à 8, dans lequel le support mis à disposition selon (i) comprend en outre 1 à 1 000 ppm de zinc et 1 à 10 000 ppm de zirconium, chacun calculés sous forme élémentaire et par rapport au poids total du support selon (i).

10. Procédé selon l'une quelconque des revendications 1 à 9, dans lequel le catalyseur contient en outre au moins un promoteur supplémentaire choisi parmi les éléments du groupe IA, VIB, VIIB et VIA du tableau périodique des éléments, de préférence choisis dans le groupe constitué par le tungstène, le lithium, le soufre, le césium, le chrome, le manganèse, le molybdène et le potassium, ledit au moins un promoteur étant de préférence appliqué à l'étape (iv) sur le support séché et/ou calciné selon (iii) par imprégnation avec le mélange G2, qui comprend en outre ledit au moins un promoteur.

11. Procédé selon l'une quelconque des revendications 1 à 10, dans lequel le catalyseur contient de l'argent en une quantité de 5 à 35 % en poids, calculée sous forme élémentaire et par rapport au poids total du catalyseur.

12. Procédé selon l'une quelconque des revendications 1 à 11, comprenant en outre
(v) le séchage et/ou la calcination du support obtenu selon (iv).

13. Catalyseur pour la fabrication d'oxyde d'éthylène, comprenant au moins de l'argent ou du zinc, appliqués sur un support, fabricable ou fabriqué par un procédé selon l'une quelconque des revendications 1 à 12.

14. Procédé de fabrication d'oxyde d'éthylène à partir d'éthylène, comprenant une oxydation d'éthylène en présence d'un catalyseur selon la revendication 13.

15. Utilisation d'un catalyseur selon la revendication 13 pour la fabrication d'oxyde d'éthylène à partir d'éthylène par oxydation d'éthylène.
